# EUROPEAN PATENT SPECIFICATION

(11) **EP 3 811 895 B1**
(45) Date of publication and mention of the grant of the patent: **23.07.2025**
(21) Application number: 19810811.0
(22) Date of filing: 20.03.2019
(51) Int. Cl.: A61C 7/36, A61F 5/56, A61C 7/08

(54) **DENTAL COUPLING MEMBER AND ORAL APPLIANCE**
ZAHNKOPPLUNGSELEMENT UND ORALES GERÄT
ÉLÉMENT DE COUPLAGE DENTAIRE ET APPAREIL ORAL

(30) Priority: 01.06.2018 JP 2018106212; 01.06.2018 JP 2018106213; 21.06.2018 JP 2018117670; 28.12.2018 JP 2018247212
(43) Date of publication of application: 28.04.2021
(73) Proprietor: Ortho Solutions, LC dba Dynaflex, Lake St. Louis MO 63367 (US)
(72) Inventor: YUKITA, Takashi, Sodegaura-shi, Chiba 299-0265 (JP); SUGITA, Haruka, Sodegaura-shi, Chiba 299-0265 (JP); HASEGAWA, Akira, Sodegaura-shi, Chiba 299-0265 (JP); TSUCHIYA, Yasufumi, Minato-ku, Tokyo 105-7122 (JP); ISHIGURO, Takehiro, Sodegaura-shi, Chiba 299-0265 (JP); ENDO, Chiaki, Chino-shi, Nagano 391-0012 (JP); KASAHARA, Kenichi, Chino-shi, Nagano 391-0012 (JP); YAZAKI Kohei, Chino-shi, Nagano 391-0012 (JP); DONUMA, Toshiaki, Chino-shi, Nagano 391-0012 (JP); SATO, You, Chino-shi, Nagano 391-0012 (JP); HAMA, Daigo, Chino-shi, Nagano 391-0012 (JP)
(74) Representative: Greaves Brewster LLP
(86) International application number: PCT/JP2019/011819
(87) International publication number: WO 2019/230152

(56) References cited:
- EP-A1- 1 712 203
- EP-A1- 1 712 203
- WO-A1-2018/074307
- DE-B4- 102007 039 761
- US-A- 5 879 157
- US-A1- 2013 280 670
- US-A1- 2014 224 257
- US-A1- 2014 224 257
- US-A1- 2014 349 243

## Description

### Technical Field

The present invention relates to a dental coupling member and an oral appliance.

### Background Art

Oral appliances (mouthpieces) are known for use in the prevention, treatment and the like of sleep apnea syndrome.

For example, Patent Literature (hereinafter, abbreviated as PTL) 1 describes a mouthpiece in which an upper jaw and a lower jaw mouthpiece are connected by a connecting member by a lower oral mouthpiece is positioned in a position to displace a lower jaw of a user wearing the mouthpiece to forward and downward directions. In PTL 1, the connecting member is rotatably attached to the upper and lower mouthpieces thereby the positions of the upper and lower mouthpieces are not fixed. According to PTL 1, the mouthpiece reduces a sense of discomfort of the user and enables a protrusion of the tongue and an ingestion of the beverage by the user.

PTL 2 describes a mouthpiece in which the connecting member has a sleeve and a rod inserted into the sleeve thereby the sleeve and the rod are connected. According to PTL 2, the length of the connecting member can be adjusted by changing the position of a connecting portion where the sleeve and the rod are connected, by a rotating nut. By adjusting the length of the connecting member, the position of the lower jaw of the user can also be freely adjusted.

PTL 3 describes a dental connecting member having an outer, a sleeve which is slidably inserted into an interior of the outer, and an inner which is screwed into the sleeve by a screw. According to PTL 3, by the length of the dental connecting member which depends on a screwing amount of the screw, the position of the lower jaw of the user can be freely adjusted.

US 2013/0280670 discloses an orthodontic correction system. The correction system attaches to a patient's dentition via an orthodontic appliance that is affixed to, e.g., pre-installed upper and lower archwires, and one or more adjustable length assemblies.

US 2014/0224257 discloses a device adapted to limit relative positioning of a mandible and a maxilla of a user that includes a housing and a telescoping member movable within the housing to change a length of the device.

EP 1 712 203 discloses an orthopaedic frame for moving the lower jaw forward that uses paired connector bars coupled to the lower and upper jaws via links. Each bar is made of two telescopic sections (6,7).

DE 10 2007 039761 discloses an orthodontic device for correcting a malposition of a lower jaw and/or a dental malposition, in particular for advancing the lower jaw, with a length-variable element which can be coupled to the lower jaw and an upper jaw.

### Citation List

### Patent Literature

PTL 1
   US Patent Application Publication No. 2007/0224567
PTL 2
   Germany Patent Application Publication No. 202012005525
PTL 3
   WO 2018/016258

### Summary of Invention

### Technical Problem

For the mouthpiece as described in PTL 1 and PTL 2, it is desired to finely adjust the position of the lower jaw to conform with the shape of the jaw or the like of the user. However, the mouthpiece described in PTL 1 has a difficulty in finely adjusting the position of the lower jaw. The mouthpiece described in PTL 2 disposes a nut at a connecting portion between the sleeve and the rod, which increases the size of the connecting member and causes a sense of discomfort to the user.

In view of the above problems, an object is to provide a dental connecting member which can easily adjust the position of the lower jaw of a user without increasing the size of the connecting member, and an oral appliance having the dental connecting member.

In addition, the lower oral advancement device described in PTL 1 and PTL 2 may accidentally shift the positions of the upper and lower mouthpieces from the optimal position due to vibration during use of the mouthpiece and the like.

In view of the above problems, another object is to provide an oral appliance capable of suppressing positional deviation of an upper jaw and a lower jaw oral appliance.

### Solution to Problem

The invention is set out in the appended claims.

A dental coupling member relating to a first aspect for solving the above problem is a dental coupling member attached to upper and lower oral appliances to couple the upper and lower oral appliances, and to regulate displacement of the lower oral appliance to rear side when a mouth is closed or opened. The dental coupling member has; an outer having a cylindrical portion which is a cylindrical member having a through hole, wherein the outer can be attached to one oral appliance of the upper and lower oral appliances; an inner having a rod-shaped insertion portion to be inserted into an interior of the cylindrical portion and slidably held by the outer, wherein the inner can be attached to the other oral appliance of the upper and lower oral appliances; and a stopper adjustably positioned inside the cylindrical portion, wherein the stopper serve to limit a slide width of the insertion portion inside the cylindrical portion by adjustment of its position along the axial direction of the cylindrical portion.

A dental coupling member relating to a second aspect for solving the above problem is a dental coupling member attached to upper and lower oral appliances to couple the upper and lower oral appliances, and to regulate displacement of the lower oral appliance to rear side when a mouth is closed or opened. The dental coupling member has: an outer having a cylindrical portion which is a cylindrical member having a through hole, wherein the outer can be attached to one oral appliance of the upper and lower oral appliances; a sleeve having a main body portion which is a cylindrical member having an insertion hole and a female screw formed on an inner surface of the insertion hole, the sleeve being slidably inserted into an interior of the through hole of the cylindrical portion; and an inner having a rod-shaped insertion portion on which a male screw which can be screwed into the female screw is formed, wherein the rod-shaped insertion portion is held by the sleeve by a threading of the male screw to the female screw, and wherein the inner can be attached to the other oral appliance of the upper and lower oral appliances. The cylindrical portion has a small diameter portion in which a diameter of the through hole is reduced. The sleeve has a pair of front and rear increased diameter portions each having a larger diameter than the small diameter portion, and when the sleeve is inserted into the interior of the cylindrical portion, one increased diameter portion of the pair of increased diameter portions is disposed on an inner side with respect to the small diameter portion, and the other increased diameter portion of the pair of increased diameter portions is disposed on an outer side with respect to the small diameter portion, respectively.

A dental coupling member relating to a third aspect for solving the above problem is a dental coupling member attached to upper and lower oral appliances to couple the upper and lower oral appliances, and to regulate displacement of the lower oral appliance to rear side when a mouth is closed or opened. The dental coupling member has: an outer having a cylindrical portion which is a cylindrical member having a through hole inside which a female screw portion is disposed, wherein the outer can be attached to one oral appliance of the upper and lower oral appliances; and an inner having a rod-shaped insertion portion on which a male screw which can be screwed into the female screw is formed, wherein the rod-shaped insertion portion is held by the outer by the threading of the male screw to the female screw, and wherein the inner can be attached to the other oral appliance of the upper and lower oral appliances. The insertion portion has an elastic portion to expand and contract the insertion portion inserted into an interior of the cylindrical portion in the axial direction of the cylindrical portion.

Further, an oral appliance according to an embodiment for solving the above problem has an upper jaw and a lower jaw oral appliance, and the dental connecting member described above, which is attached to a pair of left and right sides of the upper jaw and the lower jaw oral appliance to connect the upper jaw and the lower jaw oral appliance. Advantageous Effects

The dental connecting member can easily adjust the position of the lower jaw of the user without increasing the size of the connecting member.

### Brief Description of Drawings

The embodiments of Figs. 1 to 3C and Figs. 5A to 9C are not encompassed by the wording of the claims but are considered as useful for understanding the invention.
FIG. 1 is a schematic front view illustrating the configuration of an oral appliance;
FIG. 2A is a schematical side view showing the configuration of the oral appliance when a mouth is closed, and FIG. 2B is a schematical side view showing the configuration of the oral appliance when a mouth is opened.
FIG. 3A is a schematic side view of a dental coupling member with respect to a first embodiment.
FIG. 3B is a cross-sectional view of the dental coupling member shown in FIG. 3A showing how the inner is maximally inserted with respect to the outer.
FIG. 3C is a cross-sectional view of the dental coupling member shown in FIG. 3A showing how the inner is maximally withdrawn relative to the outer.
FIG. 4A is a cross-sectional view of a dental coupling member relating to a second embodiment showing a cross section in a side view similar to that of FIG. 3B .
FIG. 4B is a cross-sectional view of a dental coupling member relating to a second embodiment showing a cross section in a side view similar to that of FIG. 3C .
FIG. 5A is a cross-sectional view of a dental coupling member relating to a third embodiment showing a cross section in a side view similar to that of FIG. 3B .
FIG. 5B is a schematic plan view illustrating the configuration of a dental coupling member with respect to a third embodiment.
FIG. 5C is a cross-sectional view of a dental coupling member relating to a third embodiment showing a cross section in a side view similar to that of FIG. 3C .
FIG. 6A is a schematic side view of a dental coupling member with respect to a fourth embodiment.
FIG. 6B is a cross-sectional view of the dental coupling member shown in FIG. 6A showing how the inner is maximally inserted with respect to the outer.
FIG. 6C is a cross-sectional view of the dental coupling member shown in FIG. 6A showing how the inner is maximally withdrawn relative to the outer.
FIG. 7A is a schematic side view of a dental coupling member with respect to a fifth embodiment.
FIG. 7B is a cross-sectional view of the dental coupling member shown in FIG. 7A showing how the inner is maximally inserted with respect to the outer.
FIG. 7C is a cross-sectional view in a side view of the dental coupling member shown in FIG. 7A showing how the inner is maximally withdrawn relative to the outer.
FIG. 8A is a cross-sectional view in a side view schematically showing a configuration of an inner having a dental coupling member relating to a fifth embodiment.
FIG. 8B is a cross-sectional view in a side view schematically showing a configuration of an inner having a dental coupling member relating to a sixth embodiment.
FIG. 8C is a cross-sectional view in a side view schematically showing a configuration of an inner having a dental coupling member relating to a seventh embodiment.
FIG. 9A is a cross-sectional view in a side view schematically showing a configuration of an inner having a dental coupling member relating to an eighth embodiment.
FIG. 9B is a cross-sectional view in a side view schematically illustrating the configuration of another inner of the dental coupling member with respect to the eighth embodiment.
FIG. 9C is a cross-sectional view in a side view schematically illustrating a configuration of yet another inner having a dental coupling member with respect to an eighth embodiment.

### Description of Embodiments

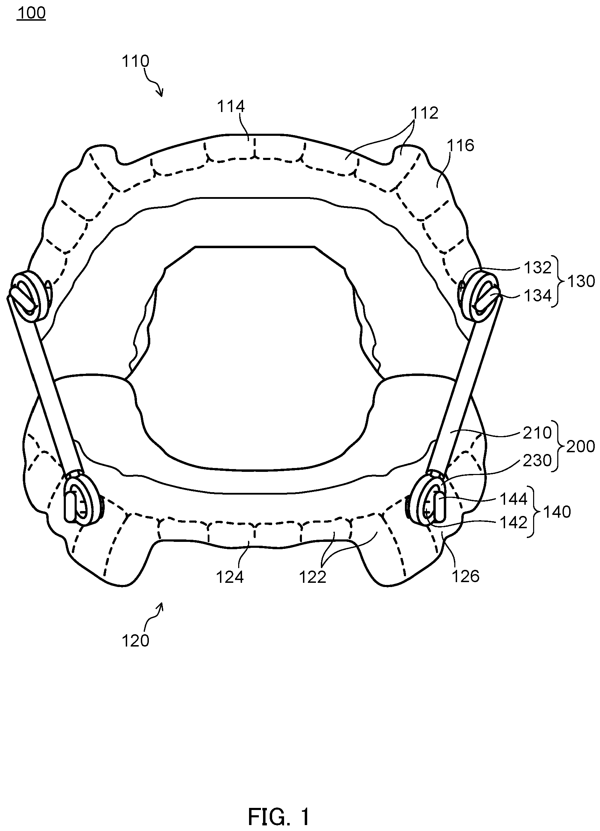

Hereinafter, a dental connecting member and an oral appliance are described with use of a plurality of embodiments. As aforementioned, the embodiments of Figs. 1 to 3C and Figs. 5A to 9C are not encompassed by the wording of the claims but are considered as useful for understanding the invention.

In the following description, "front" or "forward" and "rear" or "backward" mean a direction toward the front of the user wearing the oral appliance (direction toward the lip side as viewed from the tongue body in the mouth cavity) and a direction toward the rear side of the user (direction toward the throat side as viewed from the tongue body in the mouth cavity), and "downward" means a direction toward the left and right of the user with reference to the middle of the upper jaw wearing the oral appliance (more specifically, a direction toward each of the cheek sides as viewed from the middle of the upper jaw), "outer side" or "outside" and "inner side" or "inside" means a side close to the body surface of the user wearing the oral appliance and a side far from the body surface, respectively.

In addition, in the following description, a case in which a user wearing an oral appliance is closed is referred to as "when a mouth is closed", and a case in which a user wearing an oral appliance is open is referred to as "when a mouth is opened." When the mouth is closed, the upper oral appliance and the lower oral appliance are arranged so that the opposing surfaces of these oral appliances are substantially parallel. When the mouth is opened, the lower oral appliance is moved on a circular arc-like path, and the upper oral appliance and the lower oral appliance are arranged non-parallel so that the opposing surfaces of each other have an angle of about 45° at most.

### 1. Oral appliance

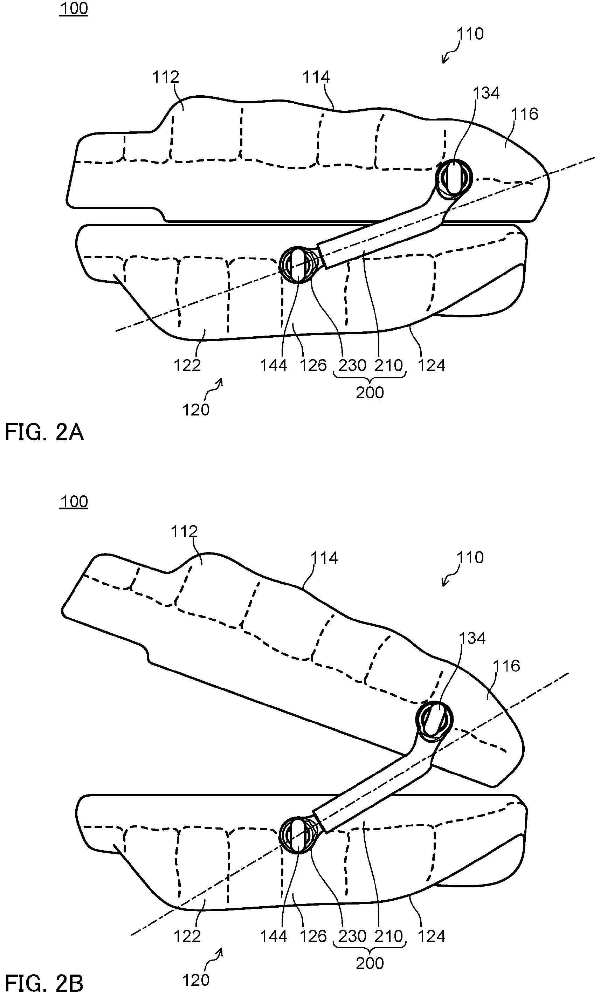

The oral appliance 100 has a pair of opposing upper and lower oral appliances and a pair of left and right dental connecting members 200 connecting the upper and lower oral appliances, as shown in FIG. 1 that is a schematic front view illustrating the configuration of the oral appliance 100, and in FIG. 2A that is a side view at the time of closing and FIG. 2B that is a side view at the time of opening. The upper oral appliance 110 is configured to be attachable to the upper jaw of the user, and the lower oral appliance 120 is configured to be attachable to the lower jaw of the user, respectively.

The upper oral appliance 110 has an upper oral appliance body 114 in which a dentition impression portion 112 wearable on a dentition of an upper law of the user, thereby upper oral appliance 110 is wearable on an upper law of the user. Also, The lower oral appliance 120 has a lower oral appliance body 124 in which a dentition impression portion 122 wearable on a dentition of a lower jaw of the user, thereby lower oral appliance 120 is wearable on a lower jaw of the user. Also, the lower oral appliance 120 is coupled to an oppositely disposed upper oral appliance 110 by a dental coupling member 200, one end of which is attached to a side surface 116 of the upper oral appliance body 114 and the other end of which is attached to a side surface 126 of the lower oral appliance body 124.

The upper oral appliance body 114 has an upper holding portion 130 on its outer surface 116 for attaching and holding an outer 210. Also, lower oral appliance body 124 has a lower holding portion 140 on its outer surface 126 for attaching and holding an inner 230. Lower holding portion 140 is disposed at a position on a front side viewed from the position of upper holding portion 130, when the mouth is closed.

Upper holding portion 130 has a substantially cylindrical upper shaft body 132 which is fixed to side surface 116 of upper oral appliance body 114 and extends outward from side surface 116, and a columnar upper flange portion 134 which extends further outwardly from the distal end of upper shaft body 132. The bottom surface of upper flange portion 134 is a rounded rectangular shape having a major axis and a minor axis, the length of the major axis is larger than the diameter of the circle constituting the bottom surface of upper shaft body 132. The long axis may have an angle of inclination of 60° to 120° with respect to an opposing surface of upper oral appliance 110 facing lower oral appliance 120. In this embodiment, the long axis is inclined to the opposing surface by about 90°.

Lower holding portion 140 has a substantially cylindrical lower shaft body 142 which is fixed to side surface 126 of lower oral appliance body 124 and extends outwardly from side surface 126, and a columnar lower flange portion 144 which extends further outwardly from the distal end of lower shaft body 142. The bottom surface of lower flange portion 144 is a rounded rectangular shape having a major axis and minor axis, the length of the major axis is larger than the diameter of the circle constituting the bottom surface of lower shaft body 142. The long axis may have an angle of inclination of 60° to 120° with respect to an opposing surface of lower oral appliance 120 facing upper oral appliance 110. In each embodiment, the long axis is inclined to the opposing surface by about 90°.

Upper oral appliance 110 and lower oral appliance 120 are made of a material which is hardly damaged by a normal occlusal force of a person.

For example, upper oral appliance 110 and lower oral appliance 120 may be made of a single hard material (for example, acrylic resin) having a Flexural modulus measured according to JIS T 6501 of 2000MPa or more and 3000MPa or less, or a material obtained by combining a soft material having a Flexural modulus of 10MPa or more and 300MPa or less and a hard material having a Flexural modulus of 1000MPa or more and 3000MPa or less.

Upper oral appliance 110 and lower oral appliance 120 may be made of a relatively soft material having a tensile strength of 150N or more and less than 2000N, preferably 150N or more and 500N or less, in order to increase the followability with respect to the teeth of the user.

Note that the tensile strength means the strength by which an upper-jaw oral appliance of an oral appliance (thickness of 3 mm) manufactured with use of a standard model of Nissin Dental Products INC. is torn when a hole of □ 1.5 mm is opened in the dentition number 6 in the upper-jaw oral appliance described above and tensile testing to the molar direction (rear side) is performed.

Examples of materials of which tensile strength is 150 N or more and less than 2000 N include olefin-based resin, polyester-based resin, urethane-based resin, polyamide-based resin, and acrylic rubber resin. Out of the above, olefin-based resin is preferred.

The olefin-based resin is a polymer obtained by homo-polymerizing olefin, or a copolymer obtained by polymerizing olefin and another monomer. As olefin, olefin of which carbon number is 2 to 6 including ethylene, propylene, butene, methyl-pentene, and hexene is preferred. Examples of other monomers include vinyl acetate.

As the olefin-based resin, polyethylene (PE), polyethylene-based resin, polypropylene (PP), polypropylene-based resin, ethylene-vinyl acetate copolymer (EVA), and the like are preferred, and polyethylene(PE), polyethylene-based resin, polypropylene (PP), polypropylene-based resin, and the like are more preferred.

The polyester-based resin is a poly-condensate of multivalent carboxylic acid (dicarboxylic acid and the like) and polyalcohol (diol and the like). Examples of the polyester-based resin include polyethylene-terephthalate (PET).

The urethane-based resin is a poly-condensate of a compound containing isocyanate groups and a compound containing hydroxyl groups. Examples of the urethane-based resin include thermoplastic polyurethane (TPU).

The polyamide-based resin is a (co)polymer obtained by bonding a large number of monomers together by amide bond. Examples of the polyamide-based resin include nylon, para-amide, and meta-amide.

The acrylic rubber resin is a (co)polymer of which main component is acrylic rubber. Examples of the acrylic resin include a block copolymer of methyl methacrylate and butyl acrylate.

As the material of which tensile strength is 150 N or more and less than 2000 N, commercially available materials such as F327 (polypropylene-based resin) made by Prime Polymer Co., Ltd. may be used.

In each of the following embodiments, a dentition impression portion which can be mounted on a dentition is formed on the upper oral appliance body and the lower oral appliance body. Note that the upper oral appliance body and the lower oral appliance body may not be of a dentition impression type as long as these oral appliance bodies have a shape to which dentition is fitted, or the upper oral appliance body and the lower oral appliance body may have the dentition impression portions in only a part of these oral appliance bodies.

The upper-jaw oral appliance and the lower-jaw oral appliance may be formed by a plurality of materials, and may be made by combining a portion formed by an organic matter and a portion formed by an inorganic matter.

### 2. Dental coupling member with respect to the first aspect

### [First Embodiment]

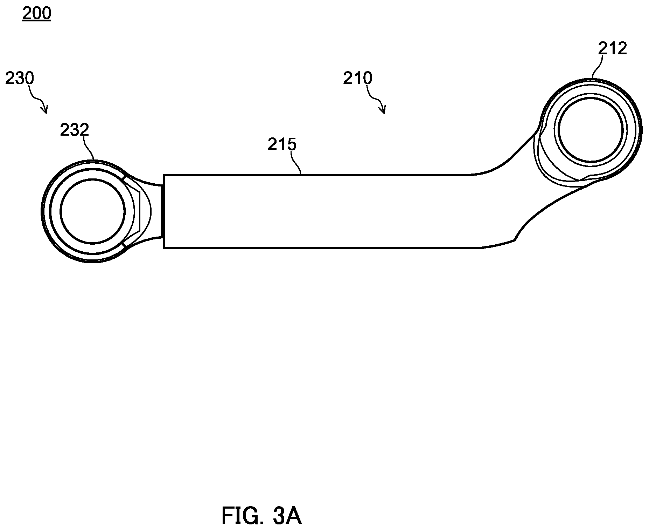

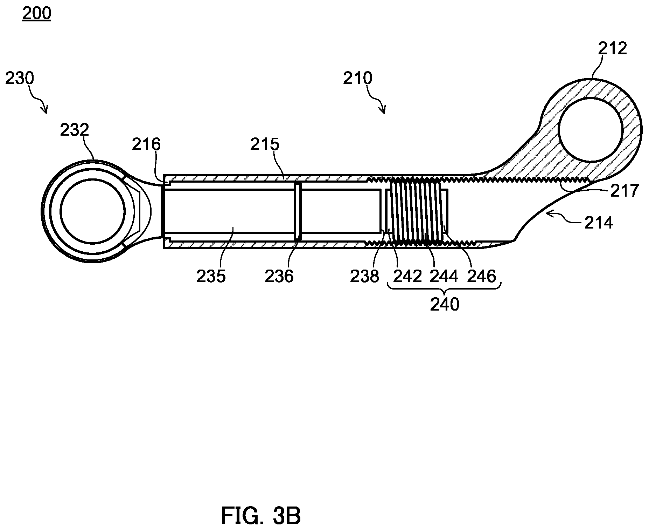

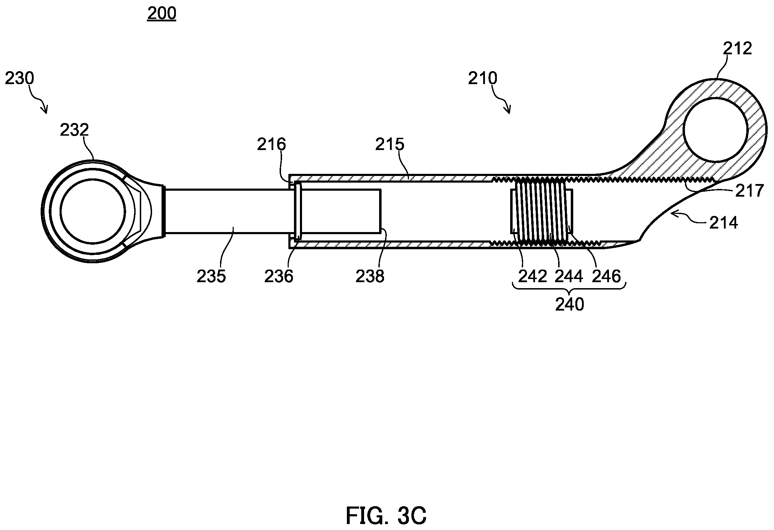

Dental coupling member 200 with respect to the first embodiment has an outer 210, an inner 230 and a stopper 240 as shown in Fig. 3A that is a schematic side view thereof, and FIGs. 3B and 3C that are cross-sectional views in the side view. Note that FIG. 3B is a cross-sectional view of the dental coupling member 200 showing a state when the inner 230 is maximally inserted with respect to the outer 210, and FIG. 3C is a cross-sectional view of the dental coupling member 200 showing a state when the inner 230 is maximally drawn with respect to the outer 210. Dental coupling member 200 slidably holds inner 230 with respect to outer 210, and thus the position of inner 230 with respect to outer 210 can be any position between FIGs. 3B and 3C . This configuration allows the dental coupling member 200 to expand and contract in a length between FIG. 3B and FIG. 3C.

Outer 210 has upper attachment portion 212 which is attached to upper oral appliance 110 and cylindrical portion 215 which has through hole 214. Upper attachment portion 212 of outer 210 is disposed on a side of cylindrical portion 215 and at a position deviated from the direction in which through hole 214 extends (hereinafter, also referred to as "axial direction of cylindrical portion 215" or simply "axial direction"). Inner 230 has lower attachment portion 232 which is attached to lower oral appliance 120 and a rod-shaped insertion portion 235 which can be slidably inserted into the interior of cylindrical portion 215 of outer 210. Lower attachment portion 232 of inner 230 is disposed substantially in the same straight line as the direction in which insertion portion 235 extends. Insertion portion 235 of inner 230 is inserted into the inside of cylindrical portion 215 of outer 210 and slidably held by outer 210, and thus outer 210 and inner 230 are coupled.

Upper attachment portion 212 of outer 210 is an eyelet having a substantially cylindrical through hole. A diameter of the bottom surface of the cylinder constituting the through hole is substantially the same as the length of the long axis of upper flange portion 134 of upper holding portion 130. The thickness of upper attachment portion 212 of outer 210 is substantially the same as the thickness of upper shaft body 132 of upper holding portion 130 (the height extending from side surface 116 of upper oral appliance body 114) or slightly thinner than the thickness of upper shaft body 132.

Lower attachment portion 232 of inner 230 is also an eyelet having a substantially cylindrical through hole. A diameter of the bottom surface of the cylinder constituting the through hole is substantially the same as the length of the long axis of lower flange portion 144 of lower holding portion 140. The thickness of lower attachment portion 232 of inner 230 is substantially the same as the thickness of lower shaft body 142 of lower holding portion 140 (the height extending from side surface 126 of lower oral appliance body 124) or slightly thinner than the thickness of lower shaft body 142.

Dental coupling member 200 is attached to upper oral appliance 110 by inserting and passing upper flange portion 134 of upper holding portion 130 into the through hole of upper attachment portion 212 of outer 210, and hooking upper attachment portion 212 of outer 210 to upper shaft body 132 of upper holding portion 130. Dental coupling member 200 is further attached to lower oral appliance 120 by inserting and passing lower flange portion 144 of lower holding portion 140 into the through hole of lower attachment portion 232 of inner 230, and hooking lower attachment portion 232 of inner 230 to lower shaft body 142 of lower holding portion 140. When the user wears the oral appliance 100, upper attachment portion 212 of outer 210 is hooked to upper shaft body 132 of upper holding portion 130, and lower attachment portion 232 of inner 230 is hooked to lower shaft body 142 of lower holding portion 140, and thus dental coupling member 200 is hardly detached from oral appliance 100.

At this time in dental coupling member 200, upper attachment portion 212 of outer 210 is rotatably hooked to upper shaft body 132 of upper holding portion 130, and lower attachment portion 232 of inner 230 is rotatably hooked to lower shaft body 142 of lower holding portion 140. Thus, oral appliance 100 is less likely to limit the opening and closing of the mouth of the user wearing the oral appliance.

Dental connecting member 200 restricts the displacement of lower oral appliance 120 to the rear side by keeping the distance between upper holding portion 130 of upper oral appliance 110 and lower holding portion 140 of lower oral appliance 120 in a predetermined range, when a mouth is closed or opened. At this time, the minimum value of the above distance defines a limit at which lower oral appliance 120 can be displaced backward.

As will be described later, dental coupling member 200 has a structure in which inner 230 is slidably coupled to outer 210 and thus expandable and contractable. In addition, inner 230 is disposed forward of outer 210 when attached to upper oral appliance 110 and lower oral appliance 120. Thus, dental coupling member 200 can reduce a sense of discomfort of the user by allowing the free movement of the lower jaw forward or downward by the user within a range in which the inner 230 can slide, while suppressing the displacement of the lower jaw of the user wearing oral appliance 100 to the rear side.

The minimum value of the distance between upper holding portion 130 and lower holding portion 140 described above is adjustable by the depth (length) that insertion portion 235 of inner 230 can be inserted with respect to cylindrical portion 215 of outer 210. At this time, by limiting the amount of sliding of insertion portion 235 inside cylindrical portion 215, the maximum insertion depth of insertion portion 235 into cylindrical portion 215 (hereinafter, simply referred to as "maximum insertion amount") can be set shallower (shorter), and thus the minimum value of the distance between upper holding portion 130 and lower holding portion 140 can be set longer. As such, it is possible to place lower jaw oral appliance 120 in a more forward direction. Alternatively, by making the maximum insertion amount deeper (longer), it is possible to shorten the minimum value of the distance between upper holding portion 130 and lower holding portion 140 so that lower jaw oral appliance 120 can be displaced more rearwardly. The above slide amount is adjusted by the position of stopper 240

Outer 210 has cylindrical portion 215 that is a cylindrical member having a substantially cylindrical through hole 214. Cylindrical portion 215 has small diameter portion 216 in which an opening diameter of through hole 214 is reduced by plastic deformation at an end portion on a side where the insertion portion 235 of the inner 230 is inserted (hereinafter, simply referred to as an "insertion side end portion"). Cylindrical portion 215 further has female screw portion 217 having a thread formed thereon on an inner peripheral surface of through hole 214 extending in an axial direction from a substantially central portion to an end portion opposite to the insertion side end portion (hereinafter, simply referred to as an "opposite side end portion").

Inner 230 has a rod-shaped insertion portion 235. Insertion portion 235 has a substantially cylindrical shape, the diameter of the circle constituting the bottom surface of the cylinder is smaller than the opening diameter of small diameter portion 216 of cylindrical portion 215. Protrusion 236 is formed on insertion portion 235. Protrusion 236 is a substantially disc-shaped member, the diameter of the circle constituting the bottom surface of the disk is smaller than the diameter of the through hole 214 of cylindrical portion 215, and larger than the opening diameter of small diameter portion 216. When the user wearing oral appliance 100 moves the lower jaw such that inner 230 slides in the direction in which the inner 230 is withdrawn from the outer 210, protrusion 236 restricts further sliding of inner 230 by abutting small diameter portion 216 of cylindrical portion 215, and thereby inhibits detachment of inner 230 from outer 210 (see FIG. 3C ).

In the present embodiment, stopper 240 is inserted into the interior of cylindrical portion 215 of outer 210, and adjustably positioned along the axial direction inside cylindrical portion 215.

Stopper 240 is a substantially cylindrical screw member, having: opposing portion 242 facing distal end 238 of insertion portion 235 of inner 230; male screw portion 244 screwed with female threaded portion 217 that cylindrical portion 215 has; and head 246 on which a screw hole that is engageable with screwdriver is formed. Opposing portion 242 and head 246 are both substantially cylindrical, the diameters of the circles constituting the bottom surfaces of the cylinders are both smaller than the diameter of through hole 214 of cylindrical portion 215, and are smaller than the distance between the threads of the screw threads disposed at a position facing through the bottom surface of female screw portion 217.

Opposing portion 242 is disposed at an end portion of stopper 240 (hereinafter, simply referred to as "facing side end portion"), on a side opposite to inner 230 when inserted into the inside of cylindrical portion 215. When the user wearing oral appliance 100 moves the lower jaw such that inner 230 slides in the direction in which the inner 230 is inserted deeper along the axial direction of cylindrical portion 215, opposing portion 242 restricts further insertion of insertion portion 235 by abutting against distal end 238 of insertion portion 235, and thereby limits the amount of sliding of inner 230. As such, stopper 240 defines the maximum insertion amount of insertion portion 235 with respect to cylindrical portion 215 (see FIG. 3B).

Male screw portion 244 is disposed between opposing portion 242 the head 246 of stopper 240. Male screw portion 244, by screwing the female screw portion 217 of cylindrical portion 215, moves stopper 240 along the axial direction of cylindrical portion 215 when stopper 240 is rotated, or fix the position of stopper 240 to cylindrical portion 215 when stopper 240 is not rotated. In this way, male screw portion 244 adjusts position of stopper 240 with respect to cylindrical portion 215.

Head 246 is disposed at an end portion (hereinafter, simply referred to as an "open side end portion") on the side of the opposite side end portion of cylindrical portion 215, which does not face inner 230 when inserted into the inside of cylindrical portion 215. Head 246 has a threaded hole in the face facing the open side end portion. By the rotation of a screwdriver engaged with the screw hole, head 246 rotates stopper 240.

With the above configuration, when rotated by a screwdriver, stopper 240 is moved inside cylindrical portion 215 while male screw portion 244 is screwed into female screw portion 217, and thus adjusted its position at a predetermined position along the axial direction of cylindrical portion 215. By the position adjustment of stopper 240, it is possible to limit the amount of sliding of insertion portion 235 inside cylindrical portion 215 to a desired degree.

Dental coupling member 200 may be made of metal materials such as titanium or alloys containing titanium, iron (such as steels including stainless steel), and alloys containing gold, silver, platinum, cobalt and chromium, as well as hard resin materials (e.g., polycarbonate resins) having a Flexural modulus as measured in accordance with JIS T 6501 of 1000MPa or more and 3000MPa or less. The metal material may be enhanced corrosion resistance by chemical conversion film.

According to the present embodiment, a smaller dental coupling member is provided.

Further, according to the present embodiment, since the amount of sliding of the insertion portion 235 can be limited in a stepless manner by the screwing of male screw portion 244 of stopper 240 and female screw portion 217 of cylindrical portion 215 of outer 210, it is possible to set the amount of advancement of lower oral appliance 120 by adapting it to the individual difference of the user.

Further, according to the present embodiment, since the maximum insertion amount of the insertion portion 235 can be set by rotation of stopper 240 while inner 230 is connected to outer 210, by insertion of thread into through hole 214, it is easy to set the advancement amount of lower oral appliance 120.

### [Second Embodiment]

The oral appliance with respect to the second embodiment differs from the oral appliance 100 of the first embodiment only in the configuration of the dental connecting member 300. Thus, the description of the configuration common to the first embodiment may be omitted hereinafter.

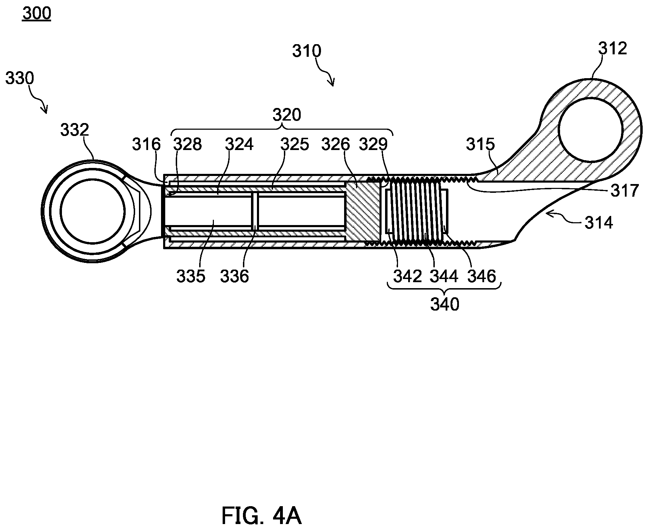

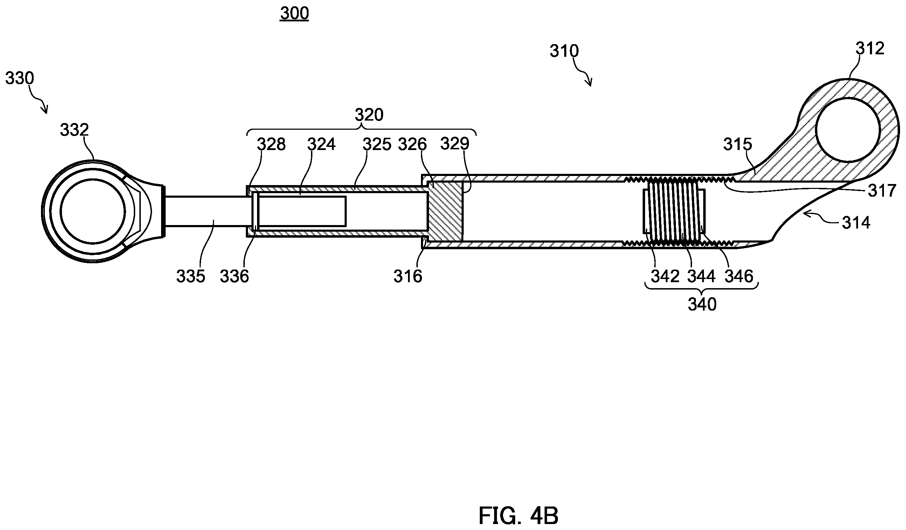

Dental coupling member 300 has outer 310, a cylindrical sleeve 320, inner 330, and stopper 340, as shown in FIG. 4A that is a cross-sectional view showing a cross-section similar to that of FIG. 3B, and FIG. 4B that is a cross-sectional view showing a cross-section similar to that of 3C.

Outer 310 has upper attachment portion 312 which is attached to upper oral appliance 110 and cylindrical portion 315 which is a cylindrical member having a substantially cylindrical through hole 314. Cylindrical portion 315 has small diameter portion 316 in which an opening diameter of through hole 314 is reduced by plastic deformation at the insertion side end portion. Cylindrical portion 315 further has female screw portion 317 having a thread formed thereon on an inner peripheral surface of through hole 314 extending in an axial direction from a substantially central portion to an end portion opposite to the insertion side end portion.

Sleeve 320 has a substantially cylindrical main body portion 325 formed at the insertion side end portion and a substantially cylindrical increased diameter portion 326 formed at the opposite side end portion. The diameter of the circle constituting the bottom surface of the cylinder of main body portion 325 is smaller than the opening diameter of small diameter portion 316 of cylindrical portion 315. The diameter of the circle constituting the bottom surface of the cylinder of increased diameter portion 326 is smaller than the diameter of through hole 314 of cylindrical portion 315 and larger than the opening diameter of small diameter portion 316. Sleeve 320 is inserted into the interior of cylindrical portion 315 of outer 310 while main body portion 325 and the increased diameter portion 326 are integrally slidable.

Main body portion 325 is a cylindrical member having a substantially cylindrical insertion hole 324. Main body portion 325 has small diameter portion 328 in which an opening diameter of insertion hole 324 is reduced by plastic deformation.

When the user wearing oral appliance 100 moves the lower jaw such that sleeve 320 slides in the direction in which sleeve 320 is withdrawn from outer 310, increased diameter portion 326 restricts further sliding of sleeve 320 by abutting small diameter portion 316 of cylindrical portion 315, and thereby inhibits further sliding of sleeve 320 (see FIG. 4B). Thus, increased diameter portion 326 prevents detachment of sleeve 320 and insertion portion 335 of inner 330 (note that in this embodiment, insertion portion 335 is slidably inserted into the inside of sleeve 320) from outer 310.

Inner 330 has lower attachment portion 332 by which inner 320 is attached to lower oral appliance 120 and a rod-shaped insertion portion 335 that is inserted into the interior of sleeve 320 and engages sleeve 320. Insertion portion 335 has a substantially cylindrical shape, the diameter of the circle constituting the bottom surface of the cylinder is smaller than the opening diameter of small diameter portion 328 of main body portion 325 of sleeve 320. Further, protrusion 336 is formed in the insertion portion 335. Protrusion 336 is a substantially disc-shaped member, the diameter of the circle constituting the bottom surface of the disk is smaller than the diameter of insertion hole 324 of main body portion 325 of sleeve 320, and larger than the opening diameter of small diameter portion 328. When the user wearing oral appliance 100 moves the lower jaw such that inner 330 is slides in the direction in which inner 330 is withdrawn from sleeve 320, protrusion 336 restricts further sliding of inner 330 by abutting small diameter portion 328 of main body portion 325, and thereby inhibits detachment of inner 330 from sleeve 320 (see FIG. 4B ). Insertion portion 335 is slidably inserted into the interior of sleeve 320 and thereby held by outer 310 into which sleeve 320 is inserted. As such, outer 310 and inner 330 are coupled.

Also in the present embodiment, stopper 340 is inserted into the interior of cylindrical portion 315 of outer 310, and adjustably positioned along the axial direction inside cylindrical portion 315.

Stopper 340 is a substantially cylindrical screw member, having: opposing portion 342 facing distal end 329 of increased diameter portion 326 of sleeve 320; male screw portion 344 screwed with female threaded portion 317 that cylindrical portion 315 has; and head 346 on which a screw hole that is engageable with screwdriver is formed. Opposing portion 342 and head 346 are both substantially cylindrical, the diameters of the circles constituting the bottom surfaces of the cylinders are both smaller than the diameter of through hole 314 of cylindrical portion 315, and are smaller than the distance between the threads of the screw threads disposed at a position facing through the bottom surface of female screw portion 317.

Opposing portion 342 is disposed on the facing side end portion of stopper 340. In this embodiment, the facing side end portion is an end portion of stopper 340 on a side facing sleeve 320 when stopper 340 is inserted into the inside of cylindrical portion 315. When the user wearing oral appliance 100 moves the lower jaw such that inner 330 slides in the direction in which insertion portion 335 is inserted deeper along the axial direction of the cylindrical portion 315, opposing portion 342 restricts further sliding of sleeve 320 by abutting distal end 329 of the increased diameter portion 326, and thereby inhibits further sliding of sleeve 320. Thus, stopper 340 limits the amount of sliding of sleeve 320 and thereby limits the amount of sliding of insertion portion 335 of inner 330 within cylindrical portion 315. Further, stopper 340 defines the maximum insertion amount of insertion portion 335 with respect to cylindrical portion 315 (see FIG. 4A).

Male screw portion 344 is disposed between opposing portion 342 and head 346 of stopper 340. Male screw portion 344, by screwing the female screw portion 317 of cylindrical portion 315, moves stopper 340 along the axial direction of cylindrical portion 315 when stopper 340 is rotated, or fix the position of stopper 340 to cylindrical portion 315 when stopper 340 is not rotated. As such, male screw portion 344 adjusts position of stopper 340 with respect to cylindrical portion 315.

Head 346 is disposed at the open side end portion of stopper 340. Head 346 has a threaded hole in the face facing the open side end portion. By the rotation of a screwdriver engaged with the screw hole, head 346 rotates stopper 340.

With the above configuration, when rotated by a screwdriver, stopper 340 is moved inside cylindrical portion 315 while male screw portion 344 is screwed into female screw portion 317, and thus adjusted its position at a predetermined position along the axial direction of cylindrical portion 315. By adjusting the position of stopper 340, the amount of sliding of sleeve 320 inside cylindrical portion 315 is also limited, and the amount of sliding of insertion portion 335 inside cylindrical portion 315 can be also limited to a desired degree.

According to this embodiment, since insertion portion 335 of inner 330 is slidably inserted into main body portion 325 of sleeve 320, the expandable and contractable length of dental coupling member 300 can be set longer. Further, according to the present embodiment, since the maximum insertion amount of the insertion portion 335 can be set by rotation of stopper 340 while inner 330 is connected to outer 310, by insertion of thread into through hole 314, it is easy to set the advancement amount of lower oral appliance 120.

### [Third embodiment]

The oral appliance according to the third embodiment differs from the oral appliance 100 of the first embodiment only in the configuration of dental connecting member 400. Therefore, the description of the configuration common to the first embodiment may be omitted hereinafter. The third embodiment is according to the claimed invention.

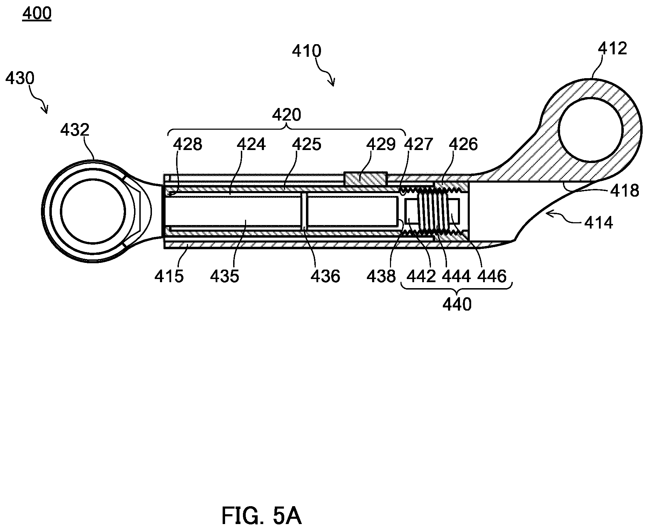

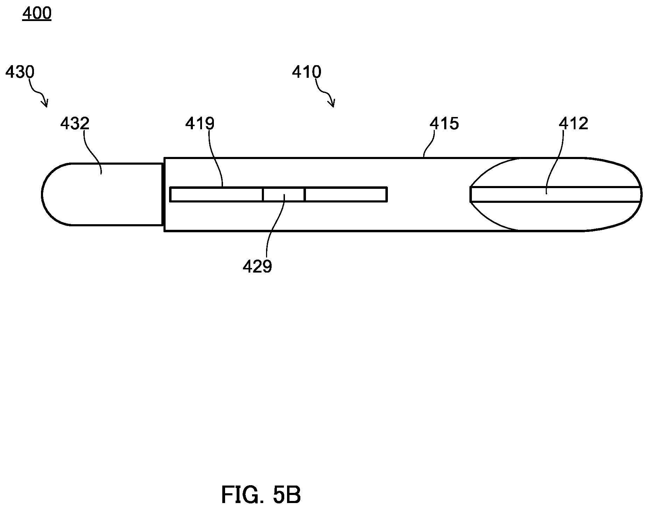

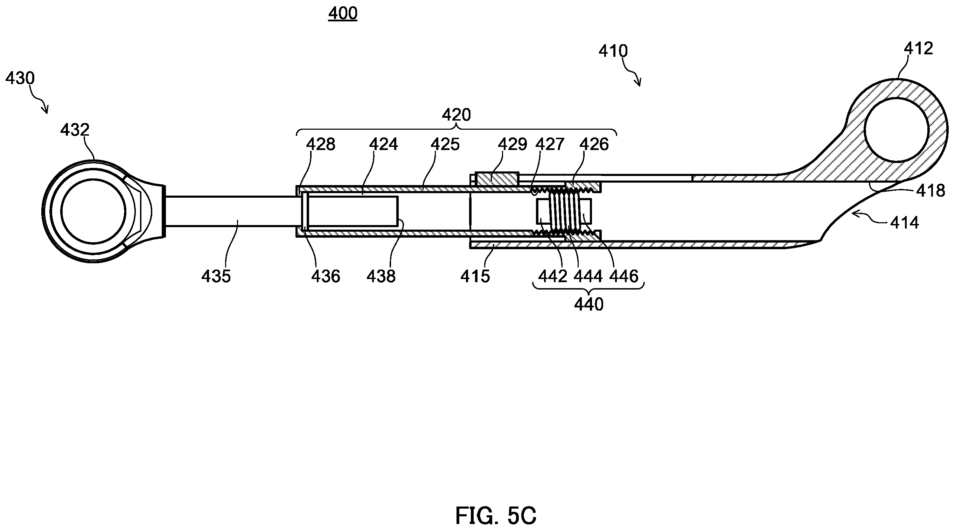

Dental coupling member 400 has outer 410, a cylindrical sleeve 420, inner 430, and stopper 440 as shown in FIG. 5A that is a cross-sectional view showing a cross-section similar to that of FIG. 3B, FIG. 5B that is a plan view, and FIG. 5C that is a cross-sectional view showing a cross-section similar to that of 3C.

Outer 410 has upper attachment portion 412 which is attached to upper oral appliance 110 and cylindrical portion 415 which is a cylindrical member having a substantially cylindrical through hole 414. In cylindrical portion 415, from at least its central portion toward the insertion side end portion, the opening diameter of the through hole 414 is substantially the same. Cylindrical portion 415 has opposite small diameter portion 418 in which an opening diameter of through hole 414 is reduced, formed at the opposite side end portion. The cylindrical portion of outer 410 has a slit 419 formed along the axial direction from insertion side end portion.

Sleeve 420 has a substantially cylindrical main body portion 425 formed at the insertion side end portion and a substantially cylindrical increased diameter portion 426 formed at the opposite side end portion. The diameter of the circle constituting the bottom surface of the cylinder of main body portion 425 is smaller than the opening diameter of through hole 414 of cylindrical portion 415. The diameter of the circle constituting the bottom surface of the cylinder of increased diameter portion 426 is smaller than the opening diameter of through hole 414 of cylindrical portion 415. Sleeve 420 is inserted into the interior of cylindrical portion 415 of outer 410 while main body portion 425 and the increased diameter portion 426 are integrally slidable.

Main body portion 425 is a cylindrical member having a substantially cylindrical insertion hole 424. Main body portion 425 has small diameter portion 428 in which an opening diameter of insertion hole 424 is reduced by plastic deformation. Main body portion 425 further has female screw portion 427 having a thread formed thereon on an inner peripheral surface of insertion hole 424 extending in an axial direction from a substantially central portion to an end portion opposite to the insertion side end portion.

Main body portion 425 further has, on its outer surface, projection 429 which can be inserted into slit 419 of cylindrical portion 415. By being inserted into the slit 419, protrusion 429 restricts the rotation of sleeve 420 inside cylindrical portion 415, and facilitates the rotation of stopper 440 for positioning stopper 440 (see FIG. 5B). Note that, when sleeve 420 slides along the axial direction of cylindrical portion 415, projection 429 slides synchronously therewith while being inserted into the slit 419. Thus, limit of the sliding of the sleeve 420 by protrusion 429 does not occur easily. When the user wearing oral appliance 100 moves the lower jaw such that sleeve 420 slides in the direction in which sleeve 420 is withdrawn from outer 410, protrusion 429 restricts further sliding of sleeve 420 by abutting the insertion side end portion of cylindrical portion 415, and thereby inhibits further sliding of sleeve 420. As such, projection 429 inhibits detachment of sleeve 420 and insertion portion 435 of inner 430 from outer 410 (see FIG. 5C).

When the user wearing oral appliance 100 moves the lower jaw such that inner 430 slides in the direction in which insertion portion 435 of inner 430 is inserted into outer 410, increased diameter portion 426 formed at the opposite side end portion of main body portion 425 restricts further sliding of sleeve 420 by abutting opposite small diameter portion 418 of cylindrical portion 415, and thereby inhibits further sliding of sleeve 420 (see FIG. 5A ). Thus, increased diameter portion 426 prevents detachment of sleeve 420 and insertion portion 435 of inner 430 (note that in this embodiment, insertion portion 435 is slidably inserted into the inside of sleeve 420) from outer 410.

Inner 430 has lower attachment portion 432 by which inner 420 is attached to lower oral appliance 120 and a rod-shaped insertion portion 435 that is slidably inserted into the interior of main body portion 425 of sleeve 420. Insertion portion 435 has a substantially cylindrical shape, the diameter of the circle constituting the bottom surface of the cylinder is smaller than the opening diameter of the small diameter portion 428 of main body portion 425 of sleeve 420. Protrusion 436 is formed on insertion portion 435. Protrusion 436 is a substantially disc-shaped member, the diameter of the circle constituting the bottom surface of the disk is smaller than the diameter of insertion hole 424 of main body portion 425 of sleeve 420, and larger than the opening diameter of small diameter portion 428. When the user wearing oral appliance 100 moves the lower jaw such that inner 430 slides in the direction in which inner 430 is withdrawn from sleeve 420, protrusion 436 restricts further sliding of inner 430 by abutting small diameter portion 428 of main body portion 425, and thereby inhibits detachment of inner 430 from sleeve 420 (see FIG. 5C). Insertion portion 435 is slidably inserted into the interior of sleeve 420 and thereby held by outer 410 into which sleeve 420 is inserted. As such, outer 410 and inner 430 are coupled.

In the present embodiment, stopper 440 is inserted into the interior of main body portion 425 of sleeve 420, and adjustably positioned along the axial direction inside main body portion 425.

Stopper 440 is a substantially cylindrical screw member, having: opposing portion 442 facing distal end 438 of insertion portion 435 of inner 430; male screw portion 444 screwed with female threaded portion 427 that main body portion 425 of sleeve 420 has; and head 446 on which a screw hole that is engageable with screwdriver is formed. Opposing portion 442 and head 446 are both substantially cylindrical, the diameters of the circles constituting the bottom surfaces of the cylinders are both smaller than the diameter of insertion hole 424 of main body portion 425, and are smaller than the distance between the threads of the screw threads disposed at a position facing through the bottom surface of female screw portion 427.

Opposing portion 442 is disposed on the facing side end portion of stopper 440. In this embodiment, the facing side end portion is an end portion of stopper 440 on a side facing insertion portion 435 of inner 430 when stopper 440 is inserted into the inside of main body portion 425. When the user wearing oral appliance 100 moves the lower jaw such that inner 430 slides in the direction in which insertion portion 435 is inserted deeper along the axial direction of the main body portion 425, opposing portion 442 restricts further sliding of inner 430 by abutting distal end 438 of insertion portion 435 of inner 430, and thereby inhibits further sliding of inner 430. Thus, stopper 440 limits the amount of sliding of inner 430 and limits the amount of sliding of main body portion 425 of sleeve 420, and thereby limits the amount of sliding of insertion portion 435 of inner 430 within cylindrical portion 415. Further, stopper 440 defines the maximum insertion amount of insertion portion 435 with respect to cylindrical portion 415 (see FIG. 5A).

Male screw portion 444 is disposed between opposing portion 442 and head 446 of stopper 440. Male screw portion 444, by screwing the female screw portion 427 of main body portion 425, moves stopper 440 along the axial direction of main body portion 425 when stopper 440 is rotated, or fix the position of stopper 440 to main body portion 425 when stopper 440 is not rotated. As such, male screw portion 444 adjusts position of stopper 440 with respect to main body portion 425 pf sleeve 420.

Head 446 is disposed at the open side end portion of stopper 440. Head 446 has a threaded hole in the face facing the open side end portion. By the rotation of a screwdriver engaged with the screw hole, head 446 rotates stopper 440.

With the above configuration, when rotated by a screwdriver, stopper 440 is moved inside main body portion 425 of sleeve 420 while male screw portion 444 is screwed into female screw portion 427, and thus adjusted its position at a predetermined position along the axial direction of main body portion 425. By adjusting the position of the stopper 440, the amount of sliding of insertion portion 435 inside main body portion 425 can be also limited to a desired degree.

According to this embodiment, the length that sleeve 420 slides inside cylindrical portion 415 by outer 410 can be set longer, and insertion portion 435 can be slidably arranged inside sleeve 420. Thus, it is possible to further reduce a sense of discomfort of the user by widening the range in which the lower jaw of the user wearing oral appliance 100 can freely move forward or downward.

Further, according to the present embodiment, since the maximum insertion amount of the insertion portion 435 can be set by rotation of stopper 440 while inner 430 is connected to outer 410, by insertion of thread into through hole 414, it is easy to set the advancement amount of lower oral appliance 120.

### [Other embodiments of the dental coupling member with respect to the first aspect]

Note that each of the above-described embodiments shows examples of a dental connecting member relating to the first aspect, and it is needless to say that the dental connecting member relating to the first aspect is not limited to the above-described embodiments.

For example, in each of the embodiments described above, the dental coupling members are attached to the oral appliance such that the inner is located anteriorly and the outer is located rearwardly, but the dental coupling member may be attached to the oral appliance such that the inner is located rearwardly and the outer is located anteriorly.

Further, in each of the embodiments described above, the dental connecting members are provided on outer surfaces of the upper and lower oral appliances, but the dental connecting member may be provided on inner surfaces of the upper and lower oral appliances.

Further, in each of the embodiments described above, the stoppers have been adjusted its position by the screwing of the male screw portions, but rather than the screwing of the screw, an elastic member such as a disc spring disposed on the outer peripheral surface of the stopper may be engaged to a projection formed on the inner peripheral surface of the cylindrical portion or the sleeve to adjust the position of the stopper.

Further, in each of the embodiments described above, the stoppers have inhibited further sliding of the inner by the abutment of the abutting portion against the end portion of the insertion portion, but, the stoppers may not be in contact with the inner, and for example, a magnet may be placed to non-contactably inhibit sliding of the inner by a magnetic force or the like.

### 3. Dental coupling member with respect to the second aspect

### [Fourth Embodiment]

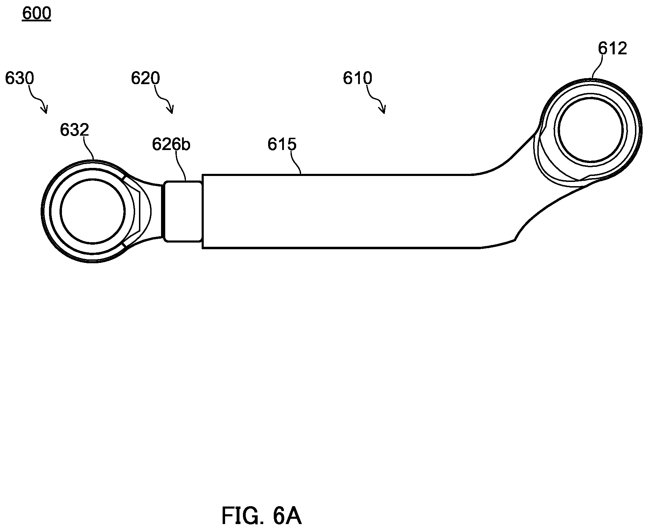

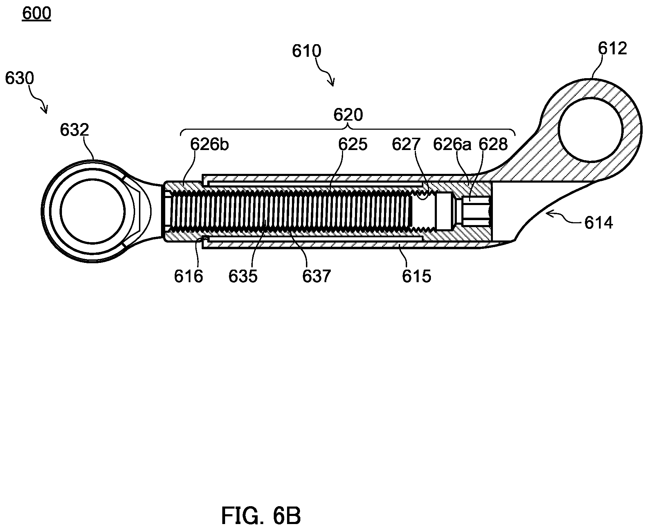

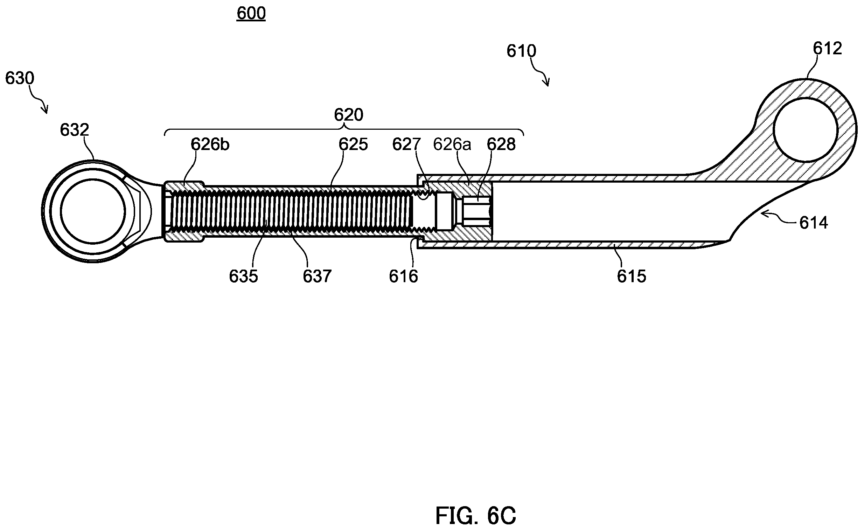

The dental coupling member 600 with respect to the present embodiment has outer 610, sleeve 620, and inner 630 as shown in Fig. 6A that is a schematic side view thereof, and FIGs. 6B and 6C that are cross-sectional views in the side view. Note that FIG. 6B is a cross-sectional view of the dental coupling member 600 showing a state when the inner 630 is maximally inserted with respect to the outer 610, and FIG. 6C is a cross-sectional view of the dental coupling member 600 showing a state when the inner 630 is maximally drawn with respect to the outer 610. Dental coupling member 600 slidably holds inner 630 with respect to outer 610, and thus the position of inner 630 with respect to outer 610 can be any position between FIGs. 6B and 6C. This configuration allows dental coupling member 600 to expand and contract in a length between FIG. 6B and FIG. 6C.

Outer 610 has upper attachment portion 612 which is attached to upper oral appliance 110 and cylindrical portion 615 which has through hole 614. Upper attachment portion 612 of outer 610 is disposed on a side of cylindrical portion 615 and at a position deviated from the direction in which through hole 614 extends (hereinafter, also referred to as "axial direction of cylindrical portion 615" or simply "axial direction"). Sleeve 620 is slidably and rotatably inserted into the interior of cylindrical portion 615 of outer 610. Inner 630 has lower attachment portion 632 which is attached to lower oral appliance 120 and a rod-shaped insertion portion 635. Lower attachment portion 632 of inner 630 is disposed substantially in the same straight line as the direction in which insertion portion 635 extends. Insertion portion 635 of inner 630 is inserted into the inside of main body portion 625 of sleeve 620 and held by sleeve 620. As such, outer 610 and inner 630 are coupled via the sleeve 620.

The description of the configuration common to the dental connection member of the first aspect may be omitted hereinafter.

As will be described later, dental coupling member 600 according to the present embodiment has a structure in which inner 630 and sleeve 620 are slidably coupled to outer 610 and thus expandable and contractable. In addition, inner 630 and sleeve 620 are disposed forward of outer 610 when attached to upper oral appliance 110 and lower oral appliance 120. Thus, dental coupling member 600 can reduce a sense of discomfort of the user by allowing the free movement of the lower jaw forward or downward by the user within a range in which the sleeve 620 can slide, while suppressing the displacement of the lower jaw of the user wearing oral appliance 100 to the rear side.

The minimum value of the distance between upper holding portion 130 and lower holding portion 140 described above is adjustable by the depth (length) that insertion portion 635 of inner 630 can be inserted with respect to cylindrical portion 615 of outer 610. The depth at which insertion portion 635 can be inserted into the portion 615 is adjusted by the depth at which insertion portion 635 of inner 630 is inserted with respect to main body portion 625 of sleeve 620.

Outer 610 has cylindrical portion 615 that is a cylindrical member having a substantially cylindrical through hole 614. Cylindrical portion 615 has small diameter portion 616 in which an opening diameter of through hole 614 is reduced by plastic deformation at an end portion on a side where the insertion portion 635 of inner 630 is inserted (hereinafter, simply referred to as an "insertion side end portion").

Sleeve 620 has a substantially cylindrical main body portion 625 formed at the insertion side end portion, and a pair of front and rear substantially cylindrical increased diameter portion 626a and an increased diameter portion 626b formed in an axial direction thereof toward an end portion opposite to the insertion side end portion (hereinafter, simply referred to as "opposite side end portion") from a substantially central portion thereof. The diameter of the circle constituting the bottom surface of the cylinder of main body portion 625 is smaller than the opening diameter of small diameter portion 616 of cylindrical portion 615. The diameter of the circle constituting the bottom surface of the cylinder of increased diameter portion 626a is smaller than the diameter of through hole 614 of cylindrical portion 615 and larger than the opening diameter of small diameter portion 616. The diameter of the circle constituting the bottom surface of the cylinder of increased diameter portion 626b is larger than the opening diameter of small diameter portion 616. Sleeve 620 is inserted into the interior of cylindrical portion 615 of outer 610 while main body portion 625, increased diameter portion 626a and increased diameter portion 626b are integrally slidable.

Main body portion 625 is a cylindrical member having an insertion hole having a shape into which insertion portion 635 of inner 630 is insertable, and main body portion 625 has a screw thread (female screw) 627 on an inner peripheral surface of the insertion hole.

When the user wearing oral appliance 100 moves the lower jaw such that sleeve 620 slides in the direction in which main body portion 625 of sleeve 620 is withdrawn from outer 610, increased diameter portion 626a restricts further sliding of sleeve 620 by abutting a surface pf opposite side end portion of small diameter portion 616 of cylindrical portion 615. As such, increased diameter portion 626a inhibits detachment of sleeve 620 from outer 610 (see FIG. 6C).

Increased diameter portion 626a has a threaded hole 628 that is engageable with a screwdriver, formed at the opposite side end portion.

When the user wearing oral appliance 100 moves the lower jaw such that sleeve 620 slides in the direction in which main body portion 625 of sleeve 620 is inserted into outer 610, increased diameter portion 626b restricts further sliding of sleeve 620 by abutting insertion side end portion of cylindrical portion 615 (including a surface of small diameter portion 616 of cylindrical portion 615 at the side of insertion side end portion). As such, increased diameter portion 626a inhibits excessive insertion of sleeve 620 into outer 610 (see FIG. 6B).

Further, as such, the length between increased diameter portion 626a and increased diameter portion 626b defines slide range of sleeve 620 (and inner 630 connected to sleeve 620).

Increased diameter portion 626b has a through hole into which insertion portion 635 of inner 630 can be inserted. The through hole has substantially the same diameter as the insertion hole of the substantially cylindrical main body portion 625, and the through hole communicates with the insertion hole.

Inner 630 has a rod-shaped insertion portion 635 that is inserted into the interior of sleeve 620 and engages sleeve 620. Insertion portion 635 has, on its outer surface, a thread (male screw) 637 which is screwed into the thread 627 formed on the inner peripheral surface of main body portion 625 of the sleeve 620. By threading thread 637 of insertion portion 635 to thread 627 of main body portion 625 of sleeve 620 and rotating sleeve 620 or inner 630, insertion portion 635 is axially aligned and thus adjusted its position with respect to sleeve 620. Insertion portion 635 is inserted into the interior of sleeve 620 and held by sleeve 620 by the screwing of the screw. Thus, sleeve 620 and the inner 630 are coupled to each other, and outer 610 and inner 630 are coupled to each other. Inner 630, while coupled with sleeve 620, integrally slides with the sleeve 620 in the interior of cylindrical portion 615 of outer 610. By the integral sliding of inner 630 and sleeve 620 inside the cylindrical portion 615, dental coupling member 600 expands and contracts.

According to the present embodiment, a smaller dental coupling member is provided.

Further, according to the present embodiment, since the depth at which inner 630 is inserted with respect to sleeve 620 can be adjusted in a stepless manner by the screwing of male screw portion 637 of inner 630 and female screw portion 627 of main body portion 625 of sleeve 620, it is possible to set the amount of advancement of lower oral appliance 120 by adapting it to the individual difference of the user.

Further, according to the present embodiment, since the depth at which inner 630 is inserted with respect to sleeve 620 can be adjusted by inserting a thread into through hole 614 and rotating sleeve 620 and adjusting the amount of screwing between male screw 637 of inner 630 and female screw 627 of main body portion 625 of sleeve 620, while inner 630 is attached to lower jaw oral appliance 120 and outer 610 is attached to upper jaw oral appliance 110. Thus, the advancing amount of lower jaw oral appliance 120 can be easily set.

Further, according to the present embodiment, the diameter of increased diameter portion 626b of sleeve 630 is larger than the diameter of through hole 614 of cylindrical portion 615. Thus, excessive insertion of sleeve 620 into outer 610 can be more reliably suppressed.

In addition, according to the present embodiment, by thickening the thicknesses of increased diameter portion 626a and increased diameter portion 626b of sleeve 620 in the axial direction, it is possible to suppress breakage of increased diameter portion 626a and increased diameter portion 626b caused by the contact of increased diameter portion 626a and increased diameter portion 626b with small diameter portion 616 at the time of sliding.

[Other embodiments of the dental coupling member with respect to the second aspect]

Note that the above-described embodiments show an example of a dental connecting member relating to the second aspect, and it is needless to say that the dental connecting member relating to the second aspect is not limited to the above-described embodiment.

For example, in the embodiment described above, the dental coupling member is attached to the oral appliance such that the inner is located anteriorly and the outer is located rearwardly, but the dental coupling member may be attached to the oral appliance such that the inner is located rearwardly and the outer is located anteriorly.

Further, in the embodiment described above, the dental connecting member is provided on outer surfaces of the upper and lower oral appliances, but the dental connecting member may be provided on inner surfaces of the upper and lower oral appliances.

Further, in each of the embodiments described above, the insertion portion of the inner has been adjusted its position by the screwing of the male screw portion, but rather than the screwing of the screw, an elastic member such as a disc spring disposed on the outer peripheral surface of the stopper may be engaged to a projection formed on the inner peripheral surface of the cylindrical portion or the sleeve to adjust the position of the insertion portion.

Further, in each of the embodiments described above, the small diameter portion of the outer and the front and rear pair of increased diameter portions of the sleeve have inhibited further sliding of the sleeve by the abutment of one of the increased diameter portions against the end portion of the small diameter portion, but, the increased diameter portions may not be in contact with the small diameter portion, and for example, a magnet may be placed to non-contactably inhibit sliding of the inner by a magnetic force or the like.

Further, in the above-described embodiment, the front and rear pair of increased diameter portions are cylindrical members each having a larger diameter than the main body portion, but, as long as it is possible to suppress further sliding of the sleeve with respect to the small diameter portion of the cylindrical portion of the outer, the increased diameter portions may be replaced by a projection or the like protruding to the outside of the main body portion of the sleeve. Also at this time, the maximum width of the bottom surface of increased diameter portion 626a disposed on the inner side of cylindrical portion 615 of outer 610 may be smaller than the diameter of through hole 614 of cylindrical portion 615 and larger than the opening diameter of small diameter portion 616. Further, the maximum width of the bottom surface, including the protrusion, of increased diameter portion 626b positioned on the outer side of cylindrical portion 615 of outer 610 may be larger than the opening diameter of the small diameter portion 616. Note that, in this specification, when simply denoted as the diameter of the increased diameter portion 626a or 626b, it means the maximum width of the bottom surface of the increased diameter portions.

Further, the diameter of the circle constituting the bottom surface of the cylinder (or the maximum width of the bottom surface including the protrusion) of increased diameter portion 626b positioned on the outer side of the sleeve may be larger than the diameter of through hole 614 of cylindrical portion 615. By this configuration, accidental penetration of increased diameter portion 626b into the inside of cylindrical portion 615 can be suppressed.

In addition, in the above-described embodiment, the small diameter portion is provided at an end portion of the cylindrical portion of the outer, but the small diameter portion does not need to be positioned at an end portion of the cylindrical portion, and can be disposed at an arbitrary place.

### 4. Dental coupling member with respect to the third aspect

### [Fifth Embodiment]

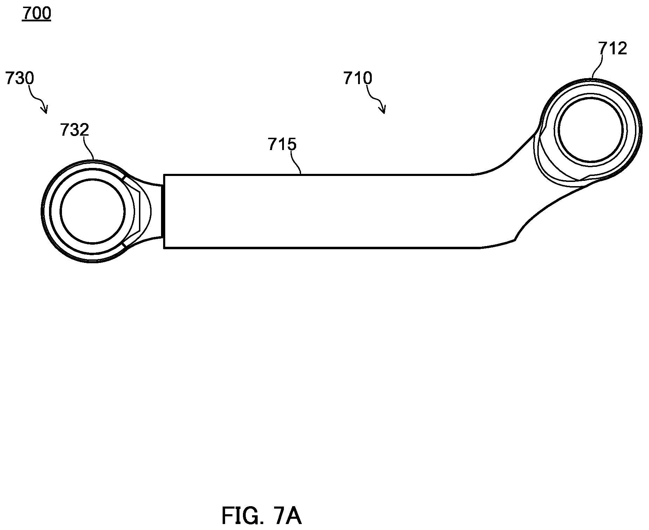

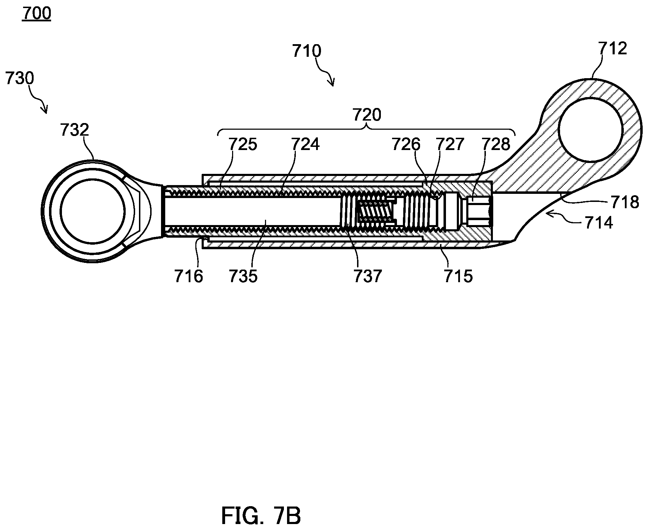

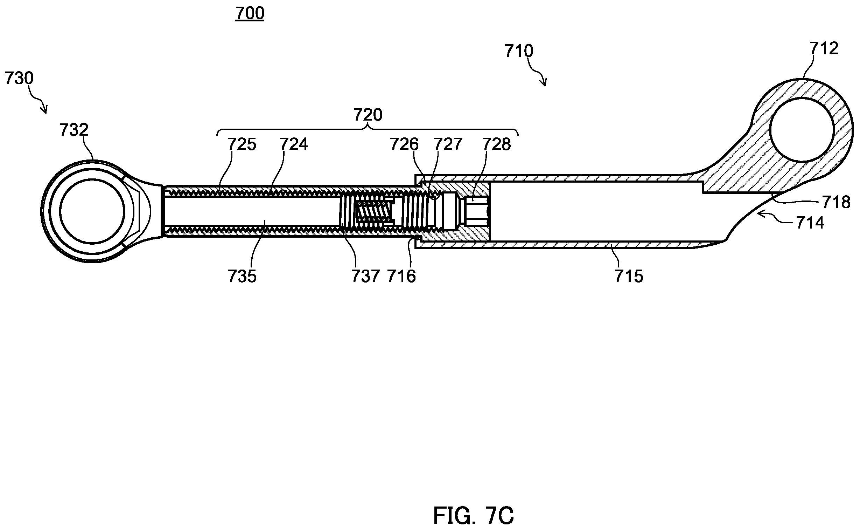

The dental coupling member 700 with respect to the present embodiment has outer 710, sleeve 720 and inner 730 as shown in a view 7A which is a schematic side view thereof and a view 7B and a view 7C which is a cross-sectional view in a side view. Note that FIG. 7B is a cross-sectional view of the dental coupling member 700 showing a state when the inner 730 is maximally inserted with respect to the outer 710, and FIG. 7C is a cross-sectional view of the dental coupling member 700 showing a state when the inner 730 is maximally drawn with respect to the outer 710. Dental coupling member 700 slidably holds inner 730 with respect to outer 710, and thus the position of inner 730 with respect to outer 710 can be any position between FIGs. 7B and 7C. This configuration allows the dental coupling member 700 to expand and contract in a length between FIG. 7B and FIG. 7C.

Outer 710 has upper attachment portion 712 which is attached to upper oral appliance 110 and cylindrical portion 715 which has through hole 714. Upper attachment portion 712 of outer 710 is disposed on a side of cylindrical portion 715 and at a position deviated from the direction in which through hole 714 extends (hereinafter, also referred to as "axial direction of cylindrical portion 715" or simply "axial direction"). Sleeve 720 is slidably inserted into the interior of cylindrical portion 715 of outer 710. Inner 730 has lower attachment portion 732 which is attached to lower oral appliance 120 and a rod-shaped insertion portion 735. Lower attachment portion 732 of inner 730 is disposed substantially in the same straight line as the direction in which insertion portion 735 extends. Insertion portion 735 of inner 730 is inserted into the inside of main body portion 725 of sleeve 720 and slidably held by sleeve 720, and thus outer 710 and inner 730 are coupled via the sleeve 720.

The description of the configuration common to the dental connection member of the first aspect and the dental connection member of the second aspect may be omitted hereinafter.

As will be described later, dental coupling member 700 of the present embodiment has a structure in which inner 730 and sleeve 720 are slidably coupled to outer 710 and thus expandable and contractable. In addition, inner 730 and sleeve 720 are disposed forward of outer 710 when attached to upper oral appliance 110 and lower oral appliance 120. Thus, dental coupling member 700 can reduce a sense of discomfort of the user by allowing the free movement of the lower jaw forward or downward by the user within a range in which the sleeve 720 can slide, while suppressing the displacement of the lower jaw of the user wearing oral appliance 100 to the rear side.

The minimum value of the distance between upper holding portion 130 and lower holding portion 140 described above is adjustable by the depth (length) that insertion portion 735 of inner 730 can be inserted with respect to cylindrical portion 715 of outer 710. The depth at which insertion portion 735 can be inserted into the portion 715 is adjusted by the depth at which insertion portion 735 of inner 730 is inserted with respect to main body portion 725 of sleeve 720.

Outer 710 has cylindrical portion 715 that is a cylindrical member having a substantially cylindrical through hole 714. Cylindrical portion 715 has small diameter portion 716 in which an opening diameter of through hole 714 is reduced by plastic deformation at an end portion on a side where the insertion portion 735 of inner 730 is inserted (hereinafter, simply referred to as an "insertion side end portion"). Cylindrical portion 715 further has an opposite small diameter portion 718 in which an opening diameter of through hole 714 is reduced, at an end portion opposite to the insertion side end portion (hereinafter, simply referred to as "opposite side end portion").

Sleeve 720 has a substantially cylindrical main body portion 725 formed at the insertion side end portion and a substantially cylindrical increased diameter portion 726 formed at the opposite side end portion. The diameter of the circle constituting the bottom surface of the cylinder of main body portion 725 is smaller than the opening diameter of small diameter portion 716 of cylindrical portion 715. The diameter of the circle constituting the bottom surface of the cylinder of increased diameter portion 726 is smaller than the diameter of through hole 714 of cylindrical portion 715, and larger than both of the opening diameter of small diameter portion 716 and the opening diameter of opposite small diameter portion 718. Sleeve 720 is inserted into the interior of cylindrical portion 715 of outer 710 while main body portion 725 and increased diameter portion 726 are integrally slidable.

Main body portion 725 is a cylindrical member having a substantially cylindrical insertion hole 724 which has a shape capable of inserting insertion portion 735 of inner 730. Main body portion 725 has a screw thread (female screw) 727 on the inner peripheral surface of the insertion hole 724 in the axial direction, toward the opposite side end portion from the insertion side end portion. In the present embodiment, thread 727 is formed with an equal pitch width.

When the user wearing oral appliance 100 moves the lower jaw such that sleeve 720 slides in the direction in which main body portion 725 of sleeve 720 is withdrawn from outer 710, increased diameter portion 726 restricts further sliding of sleeve 720 by abutting a surface pf opposite side end portion of small diameter portion 716 of cylindrical portion 715 (see FIG. 7C ). In addition, when sleeve 720 slides in the direction in which main body portion 725 of sleeve 720 is inserted into the outer 710, increased diameter portion 726 restricts further sliding of sleeve 720 by abutting opposite small diameter portion 718 of cylindrical portion 715. As such, increased diameter portion 726 inhibits detachment of sleeve 720 from outer 710 (see FIG. 7B ).

Increased diameter portion 726 has a threaded hole 728 that is engageable with a screwdriver, formed at the opposite side end portion.

Inner 730 has a rod-shaped insertion portion 735 that is inserted into the interior of sleeve 720 and engages sleeve 720. Insertion portion 735 has, on its outer surface, a thread (male screw) 737 which is screwed into the thread 727 formed on the inner peripheral surface of main body portion 725 of the sleeve 720. By threading thread 737 of insertion portion 735 to thread 727 of main body portion 725 of sleeve 720 and rotating sleeve 720 or inner 730, insertion portion 735 is axially aligned and thus adjusted its position with respect to sleeve 720. Insertion portion 735 is inserted into the interior of sleeve 720 and held by sleeve 720 by the screwing of the screw. Thus, sleeve 720 and the inner 730 are coupled to each other, and outer 710 and inner 730 are coupled to each other. Inner 730, while coupled with sleeve 720, integrally slides with the sleeve 720 in the interior of cylindrical portion 715 of outer 710. By the integral sliding of inner 730 and sleeve 720 inside the cylindrical portion 715, dental coupling member 700 expands and contracts.

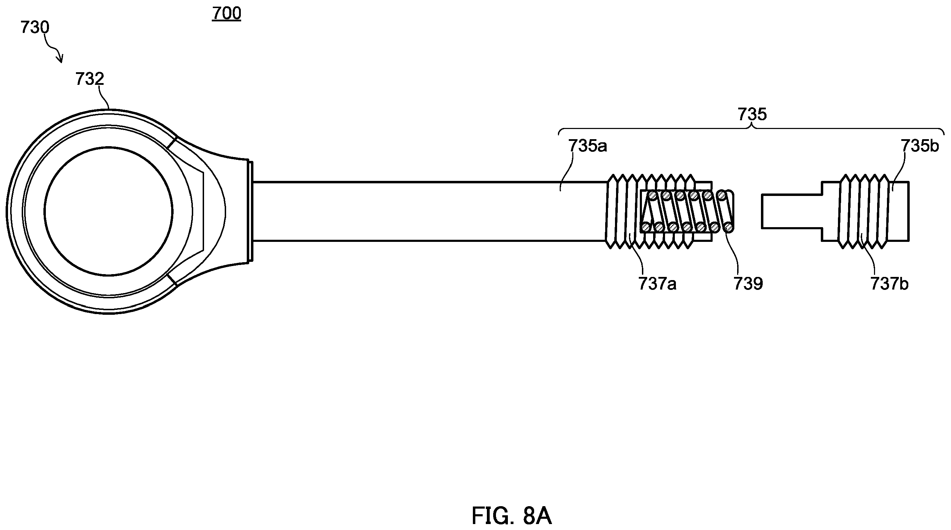

FIG 8A is a cross-sectional view in a side view schematically showing the configuration of inner 730 according to the present embodiment.

In the present embodiment, insertion portion 735 has an elastic portion for expanding and contracting insertion portion 735 in the axial direction thereof when insertion portion 735 is inserted into insertion hole 724 of main body portion 725 of sleeve 720. Specifically, insertion portion 735 has root portion 735a, distal end portion 735b, and compression coil spring 739 which is an elastic portion disposed at an intermediate portion of insertion portion 735. Root portion 735a and distal end portion 735b are connected by compression coil spring 739.

Root portion 735a is a member constituting the insertion portion 735 and is disposed on the side which is to be located on the insertion side end portion of insertion hole 724 when insertion portion 735 is inserted into insertion hole 724 of main body portion 725. Root portion 735a has thread 737a on an outer surface along an axial direction of a region on an end side close to distal end portion 735b (hereinafter, an end portion on a side close to distal end portion 735b is simply referred to as a "distal end portion", and a region on a distal end side is simply referred to as a "distal end side region"). Root portion 735a further has an invagination hole formed by invaginating into the inside from the distal end portion of the distal end side region.

Distal end portion 735b is a member disposed on the side which is to be located on the opposite side end portion of insertion hole 724 when insertion portion 735 is inserted into insertion hole 724 of main body portion 725. Distal end 735b has thread 737b throughout along the axial direction of its outer surface. Distal end portion 735b further has a protruding portion formed so as to protrude at an end portion close to root portion 735a (hereinafter, simply referred to as "root side end portion").

Compression coil spring 739 is a substantially cylindrical coil spring. Compression coil spring 739 is accommodated in an invagination hole of root portion 735a provided inside the distal region of root portion 735a. In addition, compression coil spring 739 inserts the protrusion portion of distal end portion 735b provided at the proximal end thereof into the cylindrical coil spring. As such, compression coil spring 739 connects root portion 735a and distal end portion 735b.

Compression coil spring 739 contracts insertion portion 735 so that distal end portion 735b comes closer to root portion 735a when insertion portion 735 is inserted into insertion hole 724 of main body portion 725 of sleeve 720, in a state that root portion 735a and distal end portion 735b are connected to each other. At this time, compression coil spring 739 urges root portion 735a and distal end portion 735b in a direction away from each other by the repulsive force generated by the contraction, thus causes thread 737a of root portion 735a and thread 737b of distal end portion 735b to abut against thread 727 of main body portion 725 of sleeve 720. As such, compression coil spring 739 increases the contact area between thread 737 of insert portion 735 of inner 730 and thread 727 of main body portion 725 of sleeve 720, thereby increases the torque required when sleeve 720 (thread 727) rotates relative to inner 730 (thread 737). Thus, compression coil spring 739 inhibits unintentional rotation of inner 730 (thread 737) relative to sleeve 720 (thread 727) after insertion portion 735 is inserted into insertion hole 724. Compression coil spring 739 thus can suppress unintentional misalignment of upper oral appliance 110 and lower oral appliance 120 by the rotation of sleeve 720 relative to inner 730 caused by vibration during mounting of oral appliance 100 or the like.

According to the present embodiment, a smaller dental coupling member is provided.

Further, according to the present embodiment, an unintentional positional deviation between the upper oral appliance and the lower oral appliance, due to unintentional rotation of the male screw with respect to the female screw during mounting of the oral appliance and the like, can be suppressed.

Further, according to the present embodiment, since the suppression of the change in the amount of screwing is achieved by a relatively small compression coil spring 739, it is possible to suppress the positional deviation between upper oral appliance 110 and lower oral appliance 120 without increasing the size of the oral appliance, and thus a sense of discomfort of the user is not increased.

Further, according to the present embodiment, since the depth at which inner 730 is inserted with respect to sleeve 720 can be adjusted in a stepless manner by the screwing of male screw portion 737 of inner 730 and female screw portion 727 of main body portion 725 of sleeve 720, it is possible to set the amount of advancement of lower oral appliance 120 by adapting it to the individual difference of the user.

Further, according to the present embodiment, since the depth at which inner 730 is inserted with respect to sleeve 720 can be adjusted by inserting a thread into through hole 714 and rotating sleeve 720 and adjusting the amount of screwing between male screw 737 of inner 730 and female screw 727 of main body portion 725 of sleeve 720, while inner 730 is attached to lower jaw oral appliance 120 and outer 710 is attached to upper jaw oral appliance 110. Thus, the advancing amount of lower jaw oral appliance 120 can be easily set.

### [Sixth Embodiment]

The oral appliance relating to the sixth embodiment has the same configuration as the dental connecting member and the oral appliance with respect to the fifth embodiment, except that the configuration of the insertion portion of the inner constituting the dental connecting member is different. Thus, the configuration of the dental connecting member different from that of the fifth embodiment will be described below, and a description for a common configuration will be omitted.

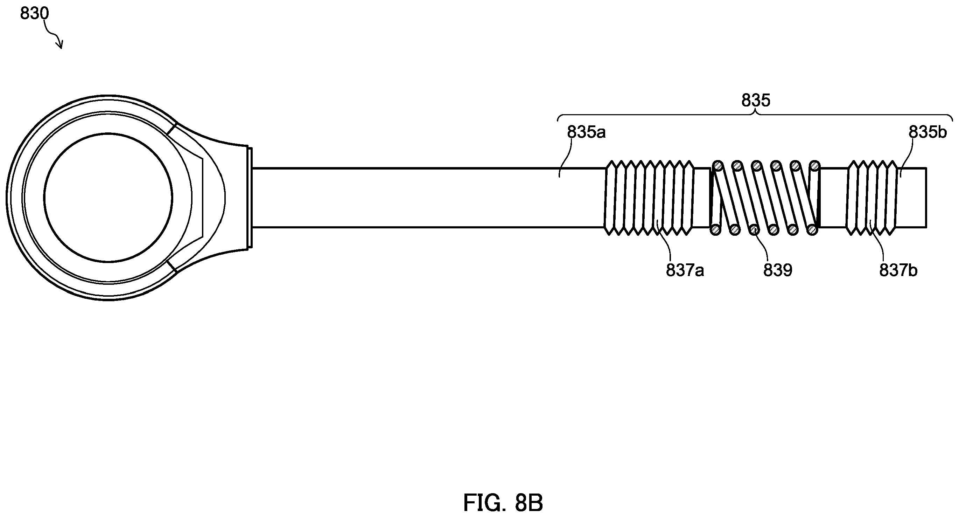

FIG 8B is a cross-sectional view in a side view schematically showing the configuration of inner 830 according to the present embodiment.

In the present embodiment, insertion portion 835 has root portion 835a, distal end portion 835b, and compression coil spring 839 which is an elastic portion disposed at an intermediate portion of insertion portion 835. Root portion 835a and distal end portion 835b are connected by compression coil spring 839.

On the other hand, in the present embodiment, root portion 835a has a protruding portion (not shown) at the distal end portion. Further, distal end portion 835b has a protruding portion (not shown) at the root side end portion.

The compression coil spring 839 is a substantially cylindrical coil spring. Compression coil spring 839 connects root portion 835a and distal end portion 835b by inserting into the inside of the cylindrical coil spring, from one end side, the protrusion formed at the distal side end of root portion 835a , and inserting, from the other end side, the protrusion formed at the root side end of distal end portion 835b.

Also in the present embodiment, compression coil spring 839 contracts insertion portion 835 so that distal end portion 835b comes closer to root portion 835a when insertion portion 835 is inserted into insertion hole 824 of main body portion 825 of sleeve 820, in a state that root portion 835a and distal end portion 835b are connected to each other. At this time, compression coil spring 839 urges root portion 735a and distal end portion 835b in a direction away from each other by the repulsive force generated by the contraction, thus causes thread 837a of root portion 835a and thread 837b of distal end portion 835b to abut against thread 827 of main body portion 825 of sleeve 820. As such, compression coil spring 839 increases the contact area between thread 837a and thread 837b of insert portion 835 of inner 830 and thread 727 of main body portion 725 of sleeve 720, thereby increases the torque required when sleeve 720 (thread 727) rotates relative to inner 830 (thread 837a and thread 837b). Thus, compression coil spring 839 inhibits unintentional rotation of inner 830 (thread 837a and thread 837b) relative to sleeve 720 (thread 727) after insertion portion 835 is inserted into insertion hole 724. Compression coil spring 839 thus can suppress unintentional misalignment of upper oral appliance 110 and lower oral appliance 120 by the rotation of sleeve 720 relative to inner 830 caused by vibration during mounting of oral appliance 100 or the like.

Further, in the present embodiment, compression coil spring 839 may also act as a male screw to be screwed against thread 727 formed on the inner surface of insertion hole 724 of main body portion 725 of sleeve 720. At this time, by setting the pitch width between the threads of compression coil spring 839 and the pitch width between the threads of threads 837a and 837b to different pitch widths, the torque required when compression coil spring 839 rotates with respect to threads 727 and the torque required when threads 837a and 837b rotate with respect to threads 727 may be different from each other. Thereby, the torque required when insertion portion 835 rotates with respect to main body portion 725 can be made larger, and unintentional positional deviation between upper oral appliance 110 and lower oral appliance 120 caused by rotation of threads 837a and 837b with respect to threads 727 due to vibration or the like during mounting of oral appliance 100 can be suppressed.

According to the present embodiment, by using the compression coil spring larger than that of the fifth embodiment, the repulsive force occurred when insertion portion 835 contracts can be increased, and the contact pressure between threads 837a and 837b of insertion portion 835 of inner 830 and threads 727 of main body portion 725 of sleeve 720 can be increased. Thus, compression coil spring 839 can more effectively suppress unintentional displacement of upper oral appliance 110 and lower oral appliance 120 due to rotation of sleeve 720 relative to inner 830 occurred by vibration during mounting of the oral appliance 100 or the like.

### [Seventh Embodiment]

The oral appliance relating to the seventh embodiment has the same configuration as the dental connecting member and the oral appliance with respect to the fifth embodiment, except that the configuration of the insertion portion of the inner constituting the dental connecting member is different. Thus, the configuration of the dental connecting member different from that of the fifth embodiment will be described below, and a description for a common configuration will be omitted.

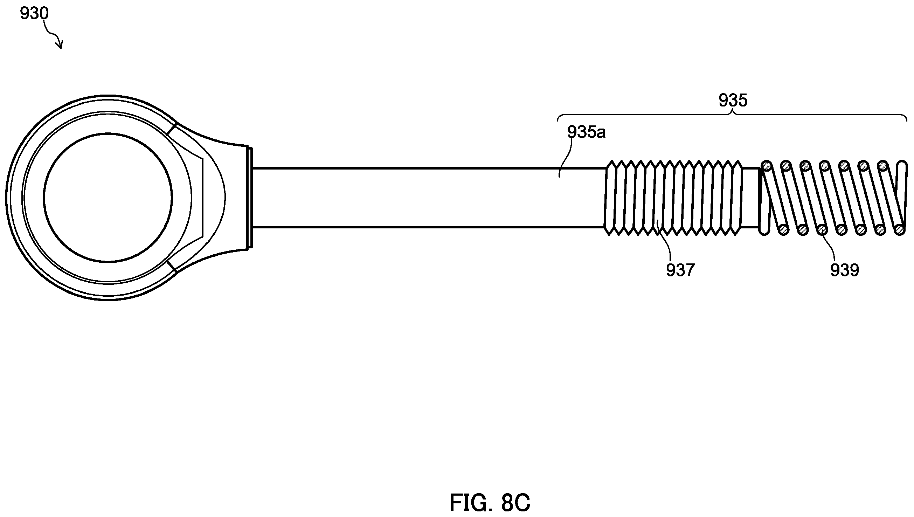

FIG 8C is a cross-sectional view in a side view showing the configuration of the inner 930 according to the present embodiment.

In the present embodiment, insertion portion 935 has root portion 935a and compression coil spring 939 which is an elastic portion disposed at the distal end of insertion portion 935.

Root portion 935a is a member constituting insertion portion 935 and is disposed on the side which is to be located on the insertion side end portion of insertion hole 724 when insertion portion 935 is inserted into insertion hole 724 of main body portion 725. Root portion 935a has threads 937 on an outer surface along an axial direction on the outer surface of the distal region.

In the present embodiment, when insertion portion 935 is inserted into insertion hole 724 of main body portion 725 of sleeve 720, compression coil spring 939 abuts against the bottom of insertion hole 724 of main body portion 725 of sleeve 720 (insertion side end portion of increased diameter portion 726), and thereby shrinks. At this time, compression coil spring 939 urges root portion 935a in the direction away from compression coil spring 939 by the repulsive force generated by the contraction, and causes thread 937 of root portion 935a to abut against thread 727 of main body portion 725 of sleeve 720. Thus, compression coil spring 939 increases the contact area between thread 937 of insert portion 935 of inner 930 and thread 727 of main body portion 725 of sleeve 720, and increases the torque required when sleeve 720 (thread 727) rotates relative to inner 930 (thread 937). Thus, compression coil spring 939 inhibits unintentional rotation of inner 930 (thread 937) relative to sleeve 720 (thread 727) after insertion portion 935 is inserted into insertion hole 724. Compression coil spring 939 thus can suppress unintentional misalignment of upper oral appliance 110 and lower oral appliance 120 by the rotation of sleeve 720 relative to inner 930 caused by vibration during mounting of oral appliance 100 or the like.

In the present embodiment, compression coil spring 939 also acts as a male thread screwed against thread 727 that main body portion 725 of sleeve 720 has on its inner surface of insertion hole 724. At this time, by setting the pitch width between the threads of compression coil spring 939 and the pitch width between the threads of thread 937 to different pitch widths, the torque required when compression coil spring 939 rotates with respect to threads 727 and the torque required when thread 937 rotate with respect to threads 727 may be different from each other. Thereby, the torque required when insertion portion 935 rotates with respect to main body portion 725 can be made larger, and unintentional positional deviation between upper oral appliance 110 and lower oral appliance 120 caused by rotation of thread 937 with respect to threads 727 due to vibration or the like during mounting of oral appliance 100 can be suppressed.

Also in the present embodiment, by using a larger compression coil spring than the fifth embodiment, the repulsive force when the insertion portion 935 is contracted is increased, the screw thread 937 and the sleeve 720 having the insertion portion 935 of the inner 930 It is also possible to increase the contact pressure between the thread 727 having the main body portion 725. Thus, compression coil spring 939 can more effectively suppress unintentional displacement of upper oral appliance 110 and lower oral appliance 120 due to rotation of sleeve 720 relative to inner 930 occurred by vibration during mounting of the oral appliance 100 or the like.

### [Eighth Embodiment]

The oral appliance relating to the eighth embodiment has the same configuration as the dental connecting member and the oral appliance with respect to the fifth embodiment, except that the configuration of the insertion portion of the inner constituting the dental connecting member is different. Thus, the configuration of the dental connecting member different from that of the fifth embodiment will be described below, and a description for a common configuration will be omitted.

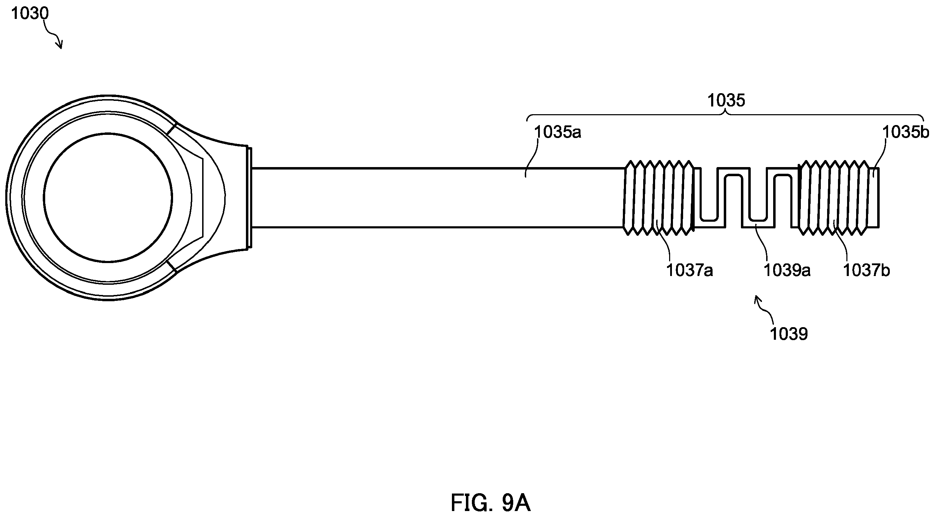

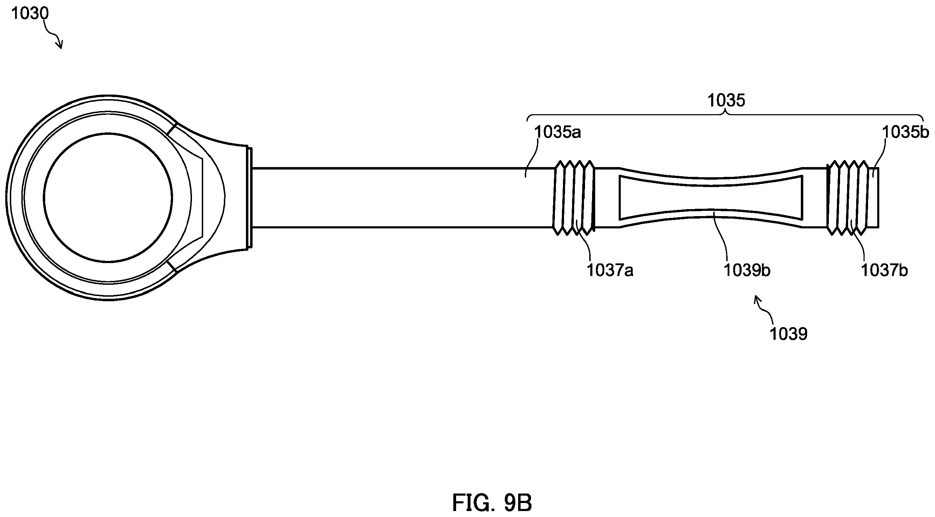

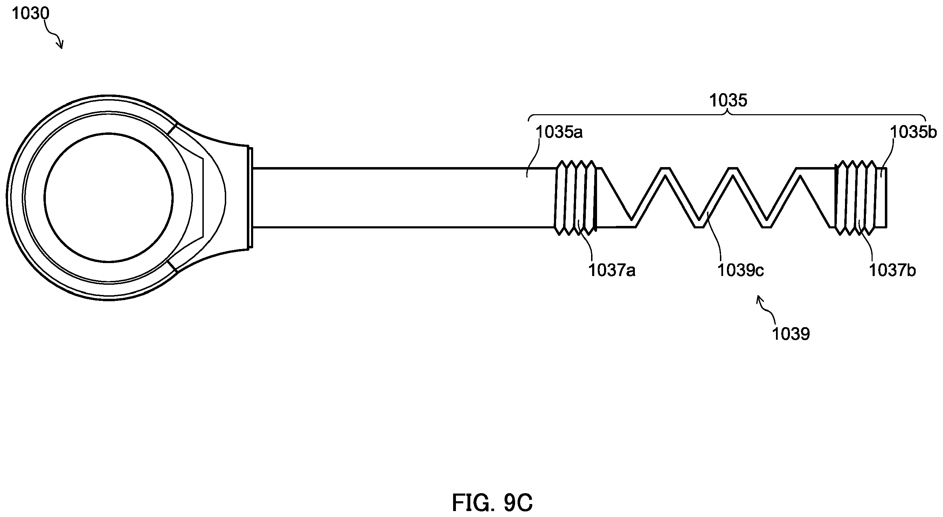

FIG. 9A, FIG. 9B and FIG. 9C are cross-sectional views in a side view illustrating the configuration of the inner 1030 with respect to the present embodiment.

In the present embodiment, insertion portion 1035 has root portion 1035a, distal end portion 1035b, and telescopic portion 1039 that is an elastic portion disposed in the intermediate portion of insertion portion 1035. Root portion 1035a and the Distal end portion 1035b is connected by telescopic portion 1039.

Telescopic portion 1039 has an elastically deformable shape, which is formed by expanding and contracting a part of portion of insertion portion 1035 to the axial direction. Telescopic portion 1039 may have a shape that connects root portion 1035a and distal end portion 1035b in a non-linear manner by a plate-like or columnar connecting member that is thinner than inner 1030, for example. More specifically, as shown in FIG. 9A, telescopic portion 1039a may have more than two straight parallel portions arranged in parallel along the axial direction of inner 1030, which are connected to each other by a connecting portion that is not parallel to these parallel portions. Otherwise, as shown in FIG. 9B, telescopic portion 1039b may have two curved parallel portions arranged parallel along the axial direction of inner 1030. Otherwise, as shown in FIG. 9C, telescopic portion 1039c may be a combination of inclined portions inclined with respect to the axial direction of inner 1030.

In the present embodiment, when insertion portion 1035 is inserted into insertion hole 724 of main body portion 725 of sleeve 720, as being a single piece in which root portion 1035a and distal end portion 1035b are integrated, at first, screw thread 1037b (male screw) is inserted into female screw 727, and then, pushing the insertion portion. Thus, distal end portion 1035b comes closer to root portion 1035a by contraction of telescopic portion 1039. At this time, telescopic portion 1039 urges root portion 1035a and distal end portion 1035b in the direction away from each other by the repulsive force generated by the contraction, and causes thread 1037a of root portion 1035a and thread 1037b of distal end portion 1035b to abut against thread 727 of main body portion 725 of sleeve 720. Thus, telescopic portion 1039 increases the contact area between threads 1037 of inserting portion 1035 of inner 1030 and threads 727 of main body portion 725 of sleeve 720, and thereby increases the torque required when sleeve 720 (threads 727) rotates relative to inner 1030 (threads 1037). Thus, telescopic portion 1039 inhibits unintentional rotation of inner 1030 (thread 1037) relative to sleeve 720 (thread 727) after insertion portion 1035 is inserted into insertion hole 724. telescopic portion 1039 thus can suppress unintentional misalignment of upper oral appliance 110 and lower oral appliance 120 by the rotation of sleeve 720 relative to inner 1030 caused by vibration during mounting of oral appliance 100 or the like.

The maximum distance from the axis of insertion portion 1035 to the outer peripheral portion of telescopic portion 1039 is smaller than diameter of root portion 1035a. Thus, when insertion portion 1035 in inserted into main body portion 725 of sleeve 720, telescopic portion 1039 does not contact with screw thread 727 of main body portion 725, and thus insertion portion 1035 can be easily inserted into main body portion 725.

### [Other embodiments of the dental coupling member with respect to the third aspect]

Note that the above-described embodiments shows an example of a dental connecting member relating to the third aspect, and it is needless to say that the dental connecting member relating to the third aspect is not limited to the above-described embodiment.

For example, in each of the embodiments described above, the dental coupling members are attached to the oral appliance such that the inner is located anteriorly and the outer is located rearwardly, but the dental coupling member may be attached to the oral appliance such that the inner is located rearwardly and the outer is located anteriorly.

Further, in each of the embodiments described above, the inner is engaged with the sleeve by screwing of the screw threads formed on the outer surface of the insertion portion of the inner to the screw thread formed on the inner surface of the insertion hole of the main body portion of the sleeve., Otherwise, the inner is engaged with the sleeve by screwing of the screw threads formed on the inner surface of the through hole of the cylindrical portion of the outer with the threads formed on the outer surface of the insertion portion of the inner. At this time, the dental connecting member does not have a sleeve and is composed of an inner and an outer.

In addition, in each of the above-described embodiments, when inserted into the inside of the insertion hole of the main body portion of the sleeve, the insertion portion of the inner is expanded and contracted by the compression coil spring. Otherwise the insertion portion may be expanded and contracted by another member having elasticity, such as a rubberlike member.

Further, in each of the embodiments described above, the dental connecting members are provided on outer surfaces of the upper and lower oral appliances, but the dental connecting member may be provided on inner surfaces of the upper and lower oral appliances.

Further, in each of the embodiments described above, the insertion portion of the inner has been adjusted its position and adjusted by the screwing of the screw thread. Otherwise, an elastic member such as a disc spring disposed on the outer peripheral surface of the insertion portion to engage with the projection formed on the inner peripheral surface of the sleeve may be used for adjusting the position of the insertion portion.

Further, in each of the above-described embodiments, the small diameter portion and the opposite side small diameter portion of the outer and the increased diameter portion of the sleeve suppressed further slide of the sleeve by abutment of the increased diameter portion against the small diameter portion or the opposite side small diameter portion. Otherwise, the increased diameter portion may not abut against the small diameter portion or the opposite side small diameter portion, and for example, a magnet may be placed to suppress the slide of the inner in a non-contact manner by a magnetic force or the like.

In addition, in each of the above-described embodiments, the small diameter portion is provided at an end portion of the cylindrical portion of the outer, but the small diameter portion does not need to be an end portion of the cylindrical portion, and can be disposed at an arbitrary place.

### Industrial Applicability

According to the dental connecting member described herein, it is possible to limit the displacement of the lower jaw to the rear side while adjusting the position of the lower jaw of the user wearing the oral appliance comprising the same. In addition, the dental connecting member is easier to adjust the amount of advancement of the lower oral appliance than in the prior art. Therefore, the dental connecting member can be used in the prevention and treatment of sleep apnea syndrome by an oral appliance comprising the same, as well as in the prevention and treatment of upper oral arthrosis and the suppression of dental bruising and the like.

### Reference Signs List

100 oral appliance
110 upper oral appliance
112 dentition impression portion
114 upper oral appliance body
116 side
120 lower oral appliance
122 dentition impression portion
124 lower oral appliance body
126 side
130 upper holding portion
132 upper shaft body
134 upper flange portion
140 lower holding portion
142 lower shaft body
144 lower flange portion
200, 300, 400 dental coupling member
210, 310, 410 outer
212, 312, 412 upper attachment portion
214, 314, 414 through hole
215, 315, 415 tube
216, 316 small diameter portion
217, 317 female screw portion
230, 330, 430 inner
232, 332, 432 lower attachment portion
235, 335, 435 insert
236, 336, 436 protrution
238, 438 distal end
240, 340, 440 stopper
242, 342, 442 opposing portion
244, 344, 444 male screw portion
246, 346, 446 head
320, 420 sleeve
324, 424 insertion hole
325, 425 main unit
326 increased diameter portion
329 distal end
418 opposite small diameter portion
419 slit
426 increased diameter portion
427 female screw portion
428 small diameter portion
429 projections
600 dental coupling member
610 outer
612 upper attachment portion
614 through hole
615 tube
616 small diameter portion
620 sleeve
625 main unit
626a, 626b increased diameter portion
627 thread
628 screw hole
630 inner
632 lower attachment portion
635 insert
637 thread
700 dental coupling member
710 outer
712 upper attachment portion
714 through hole
715 tube
716 small diameter portion
718 opposite small diameter portion
720 sleeve
724 insertion hole
725 main unit
726 increased diameter portion
727 thread
728 screw hole
730, 830, 930 inner
732 lower attachment portion
735, 835, 935, 1035 insert
735a, 835a, 935a, 1035a root
735b, 835b, 1035b distal end
737, 737a, 737b, 837a, 837b, 937a, 937b, 1037a, 1037b threads
739, 839, 939 helical compression spring
1039a, 1039b, 1039c telescopic portion

## Claims

1. A dental coupling member (300) attached to upper (110) and lower (120) oral appliances to couple the upper and lower oral appliances, and to regulate displacement of the lower oral appliance to rear side when a mouth is closed or opened, comprising:
an outer (310) having a cylindrical portion (315) which is a cylindrical member having a through hole (314), wherein the outer can be attached to one oral appliance of the upper (110) and lower (120) oral appliances;
an inner (330) having a rod-shaped insertion portion (335) to be inserted into an interior of the cylindrical portion (315) and slidably held by the outer (310), wherein the inner (330) can be attached to the other oral appliance of the upper (110) and lower (120) oral appliances; and
a stopper (340) adjustably positioned inside the cylindrical portion (315), wherein the stopper serves to limit a slide width of the rod-shaped insertion portion (335) inside the cylindrical portion (315) by adjustment of its position along the axial direction of the cylindrical portion, and
a cylindrical sleeve (320) slidably inserted into an interior of the cylindrical portion (315), wherein the rod-shaped insertion portion of the inner (330) is slidably inserted into the interior of the cylindrical sleeve (320) thereby held by the outer (310), wherein the cylindrical portion (315) has small diameter portion (316) in which an opening diameter of through hole (314) is reduced at the insertion side end portion,
the cylindrical sleeve (320) has an increased diameter portion (326), and
the increased diameter portion (326) restricts sliding of the cylindrical sleeve (320) by abutting the small diameter portion (316) of the cylindrical portion (315), and thereby inhibits detachment of the cylindrical sleeve (320) from the outer (310);
wherein the cylindrical sleeve (320) has a small diameter portion (328),
the rod-shaped insertion portion (335) of the inner (330) has a protrusion (336), and
the protrusion (336) restricts sliding of the inner (330) by abutting the small diameter portion (328) of the cylindrical sleeve (320), and thereby inhibits detachment of the inner (330) from the cylindrical sleeve (320); and
wherein the inner (330) has an attachment portion (332) which is attached to the other oral appliance, and the attachment portion (332) is disposed in the same straight line as the direction in which the rod-shaped insertion portion (335) extends;
wherein the position of the stopper (340) inside the cylindrical portion (315) is adjustable and serves to limit the slide width of the rod-shaped insertion portion (335) by limiting the slide width of the cylindrical sleeve (320) inside the cylindrical portion (315).

2. The dental coupling member (300) according to claim 1, wherein the outer (310) has a female screw portion (317) inside of the cylindrical portion (315), and the stopper (340) has a male screw portion (344) which is screwed into the female screw portion, and
wherein the position of the stopper (340) is adjustable along the axial direction of the outer (310) by a rotation of the male screw portion (344) screwed into the female screw portion (317).

3. An oral appliance (100), comprising:
upper (110) and lower (120) oral appliances, and
a pair of dental coupling members (300) according to claim 1 or 2 attached to left and right sides of the upper and lower oral appliances to connect the upper and lower oral appliances.

## Patentansprüche

1. Zahnkopplungselement (300), das an einem oberen (110) und einem unteren (120) oralen Gerät angebracht ist, um das obere und das untere orale Gerät zu koppeln und um Verschiebung des unteren oralen Geräts zu einer Rückseite zu regulieren, wenn ein Mund geschlossen oder geöffnet wird, umfassend:
ein Äußeres (310), das einen zylindrischen Abschnitt (315) aufweist, der ein zylindrisches Element ist, das ein Durchgangsloch (314) aufweist, wobei das Äußere an einem oralen Gerät von dem oberen (110) und dem unteren (120) oralen Gerät angebracht sein kann;
ein Inneres (330), das einen stabförmigen Einführungsabschnitt (335) aufweist, der in ein Inneres des zylindrischen Abschnittes (315) einzuführen ist und gleitbar durch das Äußere (310) gehalten wird, wobei das Innere (330) an dem anderen oralen Gerät von dem oberen (110) und dem unteren (120) oralen Gerät angebracht sein kann; und
einen Anschlag (340), der einstellbar innerhalb des zylindrischen Abschnittes (315) positioniert ist, wobei der Anschlag dazu dient, eine Gleitbreite des stabförmigen Einführungsabschnittes (335) innerhalb des zylindrischen Abschnittes (315) durch Einstellung seiner Position entlang der axialen Richtung des zylindrischen Abschnittes zu begrenzen, und
eine zylindrische Hülse (320), die gleitbar in ein Inneres des zylindrischen Abschnittes (315) eingeführt ist, wobei der stabförmige Einführungsabschnitt des Inneren (330) gleitbar in das Innere der zylindrischen Hülse (320) eingeführt ist, wodurch er durch das Äußere (310) gehalten wird, wobei der zylindrische Abschnitt (315) einen Abschnitt mit kleinem Durchmesser (316) aufweist, in dem ein Öffnungsdurchmesser des Durchgangslochs (314) an dem Einführungsseitenendabschnitt reduziert ist,
wobei die zylindrische Hülse (320) einen Abschnitt mit erhöhtem Durchmesser (326) aufweist, und
der Abschnitt mit erhöhtem Durchmesser (326) Gleiten der zylindrischen Hülse (320) beschränkt, indem er an dem Abschnitt mit kleinem Durchmesser (316) des zylindrischen Abschnittes (315) anliegt und dadurch Ablösen der zylindrischen Hülse (320) von dem Äußeren (310) verhindert;
wobei die zylindrische Hülse (320) einen Abschnitt mit kleinem Durchmesser (328) aufweist,
der stabförmige Einführungsabschnitt (335) des Inneren (330) einen Vorsprung (336) aufweist und
der Vorsprung (336) Gleiten des Inneren (330) beschränkt, indem er an dem Abschnitt mit kleinem Durchmesser (328) der zylindrischen Hülse (320) anliegt und dadurch Ablösen des Inneren (330) von der zylindrischen Hülse (320) verhindert; und
wobei das Innere (330) einen Anbringungsabschnitt (332) aufweist, der an dem anderen oralen Gerät angebracht ist, und der Anbringungsabschnitt (332) in der gleichen geraden Linie wie die Richtung angeordnet ist, in der sich der stabförmige Einführungsabschnitt (335) erstreckt;
wobei die Position des Anschlags (340) innerhalb des zylindrischen Abschnittes (315) einstellbar ist und dazu dient, die Gleitbreite des stabförmigen Einführungsabschnittes (335) zu begrenzen, indem die Gleitbreite der zylindrischen Hülse (320) innerhalb des zylindrischen Abschnittes (315) begrenzt wird.

2. Zahnkopplungselement (300) nach Anspruch 1, wobei das Äußere (310) einen weiblichen Schraubenabschnitt (317) innerhalb des zylindrischen Abschnittes (315) aufweist und der Anschlag (340) einen männlichen Schraubenabschnitt (344) aufweist, der in den weiblichen Schraubenabschnitt geschraubt ist, und
wobei die Position des Anschlags (340) entlang der axialen Richtung des Äußeren (310) durch eine Drehung des männlichen Schraubenabschnittes (344), der in den weiblichen Schraubenabschnitt (317) geschraubt ist, einstellbar ist.

3. Orales Gerät (100), umfassend:
ein oberes (110) und ein unteres (120) orales Gerät, und
ein Paar Zahnkopplungselemente (300) nach Anspruch 1 oder 2, die an der linken und der rechten Seite des oberen und des unteren oralen Geräts angebracht sind, um das obere und das untere orale Gerät zu verbinden.

## Revendications

1. Élément de couplage dentaire (300) fixé aux appareils buccaux supérieur (110) et inférieur (120) destiné au couplage des appareils buccaux supérieur et inférieur, et destiné à réguler le déplacement de l'appareil buccal inférieur vers le côté arrière lorsqu'une bouche est fermée ou ouverte, comprenant :
une partie extérieure (310) comportant une partie cylindrique (315) qui est un élément cylindrique comportant un trou traversant (314), ladite partie extérieure pouvant être fixée à un appareil buccal des appareils buccaux supérieur (110) et inférieur (120) ;
une partie intérieure (330) comportant une partie d'insertion en forme de tige (335) destinée à être insérée à l'intérieur de la partie cylindrique (315) et maintenue de manière coulissante par la partie extérieur (310), ladite partie intérieure (330) pouvant être fixée à l'autre appareil buccal des appareils buccaux supérieur (110) et inférieur (120) ; et
une butée (340) positionnée de manière réglable à l'intérieur de la partie cylindrique (315), ladite butée servant à limiter une largeur de coulissement de la partie d'insertion en forme de tige (335) à l'intérieur de la partie cylindrique (315) par réglage de sa position le long de la direction axiale de la partie cylindrique, et
un manchon cylindrique (320) inséré de manière coulissante à un intérieur de la partie cylindrique (315), ladite partie d'insertion en forme de tige de la partie interieur (330) étant insérée de manière coulissante à l'intérieur du manchon cylindrique (320) ainsi maintenu par la partie extérieur (310), ladite partie cylindrique (315) comportant une partie de petit diamètre (316) dans laquelle le diamètre d'une ouverture du trou traversant (314) est réduit au niveau de la partie d'extrémité côté insertion,
ledit manchon cylindrique (320) comportant une partie de diamètre accru (326), et
ladite partie de diamètre accru (326) limitant le coulissement du manchon cylindrique (320) en venant en butée contre la partie de petit diamètre (316) de la partie cylindrique (315), et empêchant ainsi le détachement du manchon cylindrique (320) de la partie extérieure (310) ;
ledit manchon cylindrique (320) comportant une partie de petit diamètre (328),
ladite partie d'insertion en forme de tige (335) de la partie intérieure (330) comportant une saillie (336), et
ladite saillie (336) limitant le coulissement de la partie intérieure (330) en venant en butée contre la partie de petit diamètre (328) du manchon cylindrique (320), et empêchant ainsi le détachement de la partie intérieure (330) du manchon cylindrique (320) ; et
ladite partie intérieure (330) comportant une partie de fixation (332) qui est fixée à l'autre appareil buccal, et ladite partie de fixation (332) étant disposée dans la même ligne droite que la direction dans laquelle la partie d'insertion en forme de tige (335) s'étend ;
ladite position de la butée (340) à l'intérieur de la partie cylindrique (315) étant réglable et servant à limiter la largeur de coulissement de la partie d'insertion en forme de tige (335) en limitant la largeur de coulissement du manchon cylindrique (320) à l'intérieur de la partie cylindrique (315).

2. Élément de couplage dentaire (300) selon la revendication 1, ladite partie extérieure (310) comportant une partie de vis femelle (317) à l'intérieur de la partie cylindrique (315), et ladite butée (340) comportant une partie de vis mâle (344) qui est vissée dans la partie de vis femelle, et
ladite position de la butée (340) étant réglable le long de la direction axiale de la partie extérieure (310) par une rotation de la partie de vis mâle (344) vissée dans la partie de vis femelle (317).

3. Appareil buccal (100), comprenant :
des appareils buccaux supérieur (110) et inférieur (120), et
une paire d'éléments de couplage dentaire (300) selon la revendication 1 ou 2 fixés aux côtés gauche et droit des appareils buccaux supérieur et inférieur de manière à raccorder les appareils buccaux supérieur et inférieur.
